(19) Europäisches Patentamt
European Patent Office
Office européen des brevets

(11) EP 4 417 195 A1

(12) EUROPEAN PATENT APPLICATION

(43) Date of publication:
**21.08.2024 Bulletin 2024/34**

(21) Application number: **23156479.0**

(22) Date of filing: **14.02.2023**

(51) International Patent Classification (IPC):
***A61K 9/20*** *(2006.01)* ***A61K 31/00*** *(2006.01)*
***A61K 47/26*** *(2006.01)* ***A61K 47/32*** *(2006.01)*
***A61P 9/12*** *(2006.01)*

(52) Cooperative Patent Classification (CPC):
**A61K 9/2095; A61K 9/2018; A61K 9/2027; A61K 31/00; A61K 47/26; A61K 47/32; A61P 9/12; B29C 64/00; B33Y 30/00; B33Y 80/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Universitätsklinikum Hamburg-Eppendorf (UKE)**
**20246 Hamburg (DE)**

(72) Inventors:
- **GUTOWSKI, Tobias**
  **20246 Hamburg (DE)**
- **ROSCH, Moritz**
  **20246 Hamburg (DE)**
- **DADKAH, Adrin**
  **20246 Hamburg (DE)**
- **EGGERT, Jan**
  **20246 Hamburg (DE)**

(74) Representative: **Stüven, Ralf**
**RGTH Patentanwälte PartGmbB**
**Kirchenhang 32b**
**21073 Hamburg (DE)**

# (54) EXCIPIENT COMPOSITION FOR THE PREPARATION OF AMORPHOUS SOLID DISPERSONS/SOLUTIONS

(57) The invention provides an excipient composition for the preparation of an amorphous solid dispersion or amorphous solid solution suitable for, i.a., 3D printing of immediate release tablets. The excipient composition comprises:
a) 8-20 % by weight of the excipient composition of sugar alcohol, and
b) 80-92 % by weight of the excipient composition of a polymer blend, the polymer blend comprising a first polymer component and a second polymer component in a weight ratio of 1:0.55 - 1:1.75, the first polymer component being a copolymer of 1-vinyl-2-pyrrolidone and vinyl acetate, and the second polymer component being polyethylene glycol-polyvinyl alcohol graft copolymer.

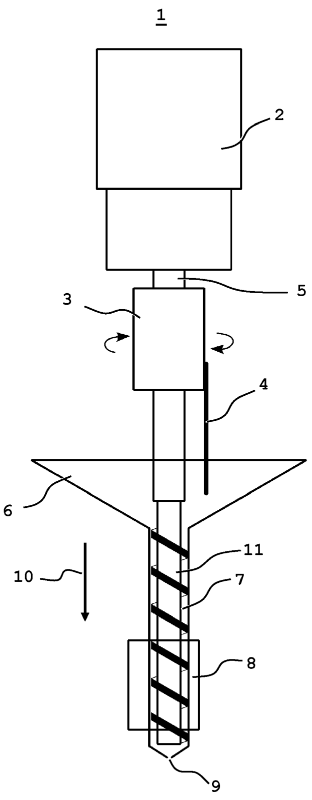


Fig. 1

EP 4 417 195 A1

## Description

The invention relates to an excipient composition for the preparation of amorphous solid dispersions/solutions. In further aspects, the invention relates, inter alia, to a pharmaceutical composition comprising the excipient composition and a solid dosage form manufactured using the pharmaceutical composition. The excipient composition can, for example, be used for the additive manufacturing, in particular direct powder extrusion printing, of a pharmaceutical solid dosage form.

In the course of the increasing individualization of medical treatments and drug dosing regimens, sometimes termed "precision medicine" or "personalized medicine", the use of additive manufacturing (AM), also called "3D printing" (3DP), has gained more and more interest, in particular in view of a decentralized on-site manufacturing in hospitals and pharmacies. Additive manufacturing is a computer-controlled process creating three-dimensional objects by depositing or joining suitable materials, usually in layers. Several individual methods of additive manufacturing are known, for example binder jetting (BJ), fused deposition modelling (FDM), direct powder extrusion (DPE), selective laser sintering (SLS) and semi-solid extrusion (SSE). Some of these technologies have already been used in the pharmaceutical field, for example, for the manufacture of solid oral dosage forms like tablets, sometimes called "printlets" (see, e.g., Zhang et al. 2018; Gioumouxouzis et al. 2019; Varghese et al. 2022; Auriemma et al. 2022; Mathew et al. 2020; Pitzanti et al. 2022; Serrano et al. 2023). Binder jetting, for example, has been used for the currently only 3D printed drug product (Spritam®, Aprecia Pharmaceuticals LLC, Hong et al. 2021) that has been approved by the U.S. Food and Drug Administration (FDA).

Current pharmaceutical literature on material extrusion techniques is focused on FDM (Windolf et al. 2021, Windolf et al. 2022; Chamberlain et al. 2022; Kissi et al. 2021; Goyanes et al. 2015) and SSE (Tagami et al. 2021; Yu et al. 2020; Seoane-Viaño et al. 2021; Yan et al. 2020). Both technologies are readily available and offer the possibility of producing relatively small batches, which minimizes the amounts of active pharmaceutical ingredient (API) and excipients needed to conduct formulation studies, for example (see, e.g., Auriemma et al. 2022). FDM printing requires an extruder to manufacture the filament that serves as a feedstock in the printing process. In contrast, SSE, also known as "pressure-assisted microsyringe", PAM, uses pre-filled syringes, from which a gel or paste is extruded, meaning that SSE is a discontinuous process.

The use of direct powder extrusion (DPE) has also been reported for the manufacturing of 3D printed tablets (Goyanes et al. 2019; Sánchez-Guirales et al. 2021; Malebari et al. 2022; Mendibil et al. 2021; Boniatti et al. 2021). DPE can be designed as a continuous process and does not require a complex and time-consuming preceding feedstock preparation. Moreover, other than FDM, for example, the DPE can be used with only a few grams of formulation. It has also been reported, however, that the powder flow considerably influences the DPE printing process (Boniatti et al. 2021).

Amorphous solid dispersions (ASDs) or amorphous solid solutions are usually polymer-based compositions that are frequently used in drug products for enhanced dissolution and bioavailability of active pharmaceutical ingredients (see, for example, Pandi et al. 2020; Bhujbal et al. 2021). These compositions are particularly useful for drug products including poorly water-soluble drugs.

It is an object of the invention to improve the preparation and the characteristics of personalized drug products.

In a first aspect, the invention provides an excipient composition for the preparation of an amorphous solid dispersion or amorphous solid solution, the excipient composition comprising:

a) 8-20 % by weight of the excipient composition of sugar alcohol, and
b) 80-92 % by weight of the excipient composition of a polymer blend, the polymer blend comprising a first polymer component and a second polymer component in a weight ratio of 1:0.55 - 1:1.75, the first polymer component being a copolymer of 1-vinyl-2-pyrrolidone and vinyl acetate, and the second polymer component being polyethylene glycol-polyvinyl alcohol graft copolymer.

The invention provides an excipient composition that can advantageously be used in the preparation of a drug product, in particular a drug product based on an amorphous solid dispersion or amorphous solid solution in the preparation. The excipient composition of the invention is especially useful in the preparation of personalized medications. It can, for example, be used in additive manufacturing, in particular in direct powder extrusion printing, of solid dosage forms or the preparation of high dose dry powder formulations by spray drying. In particular, the excipient composition of the invention can be used for the preparation of an immediate release formulation with excellent hardness values that allows consistent 3D-printing, e.g., of tablets.

The term "amorphous solid dispersion", ASD, refers to a solid dispersion in which an amorphous active pharmaceutical ingredient (API), in particular an API of poor aqueous solubility, is dispersed within an excipient matrix, usually a polymer matrix, in order to enhance solubility and dissolution of the API (see, Pandi et al. 2020; Bhujbal et al. 2021; S'ari et al. 2021). In case of an "amorphous solid solution" the API/polymer combination is completely phase-mixed (see, e.g., Nagapudi, K. & Jona, J. 2008).

The term "pharmaceutical composition" relates to a composition, i.e., a mixture or blend of substances, suitable for administering to an individual, which comprises a pharmaceutically effective amount of an active pharmaceutical ingredient (API) and suitable excipients.

The term "active pharmaceutical ingredient", API, relates to a substance used as an ingredient in a pharmaceutical composition that is responsible for or intended to exert or convey a desired pharmacological effect, e.g., a beneficial health effect in an individual to which the composition is administered. The terms "pharmaceutically active agent", "pharmacologically active substance", "drug substance" or "active ingredient" may, for example, also be used synonymously.

The term "excipient composition" relates to a composition, i.e., a mixture or blend, of excipients.

The term "polymer blend" relates to a polymer composition, i.e., an intimate mixture or blend of at least two different polymers. As used herein, terms like "the polymer blend as described herein" or "the polymer blend as described above" relates to a polymer blend comprising, preferably being composed of, a first polymer component and a second polymer component in a weight ratio of x:y, the first polymer component being a copolymer of 1-vinyl-2-pyrrolidone and vinyl acetate, and the second polymer component being polyethylene glycol-polyvinyl alcohol graft copolymer. The weight ratio x:y is preferably 1:0.55 - 1: 1.75, more preferably 1:0.65 - 1:1.7, 1:0.75 - 1:1.7, 1:0.85 - 1:1.65, 1:0.9 - 1:1.6, 1:0.95 - 1:1.5, 1:1.0 - 1:1.4, 1:1.05 - 1:1.3, 1:1.1 - 1:1.3, 1:1.15 - 1:1.25, or 1:1.15 - 1:1.2, for example 1.2.

The term "excipient" relates to any substance other than the active pharmaceutical ingredient (API) intentionally included in a pharmaceutical composition. An excipient is preferably, but not necessarily, an essentially pharmacologically inert (pharmacologically inactive) and non-toxic substance. Excipients can, for example, be included in a pharmaceutical composition in order to protect, support, or enhance stability, bioavailability, or patient acceptability of the API or pharmaceutical composition, to aid in the processing of the API during its manufacture, to assist in the effectiveness and/or delivery of the pharmaceutical composition, or to assist in maintaining the integrity of the pharmaceutical composition during storage. Examples are plasticizers, adjuvants, antiadherents, binders, disintegrants, glidants, lubricants, humectants, buffers, adhesives, thickeners, preservatives, coatings, fillers, diluents, flavouring agents, colorants, sweeteners etc.

The term "sugar alcohol" ("polyhydric alcohol", "alditol") relates to organic compounds having the general formula $HOCH_2(CHOH)_nCH_2OH$, n being, for example, 4-12, e.g., 4, 5 or 6. Sugar alcohols are typically derived from sugars, and having no more than one hydroxy group attached to each carbon atom. Examples are mannitol and sorbitol.

The term "solid dosage form" relates to a solid, i.e., non-liquid and non-gaseous, pharmaceutical dosage form. A solid dosage form may, for example be intended for oral administration. Examples of solid dosage forms are, for example, tablets, capsules, powders, granules, suppositories. A common solid dosage form for oral use (solid oral dosage form, SODF) is a tablet or capsule. The term "printlet" may be used for a tablet manufactured by a 3D-printing process, e.g., direct powder extrusion printing.

The term "immediate release formulation" relates to dosage forms, e.g., a tablet, that disintegrate and release the active pharmaceutical ingredient without intended delay. An immediate release formulation may, for example, be a formulation showing an in-vitro dissolution of $\geq$85 of the active ingredient within 30 min (see, for example, Verbeeck & Musuamba 2012) or an in-vitro dissolution of >80% in 45 min (Ph. Eur. 10.6/5.17). In contrast, a "sustained release formulation" or "delayed release formulation" is designed to delay and/or prolong dissolution and absorption of the active ingredient in a controlled manner.

The term "additive manufacturing", AM, also called "3D printing", 3DP, relates to a computer-controlled process in which three-dimensional objects are formed by depositing or fusing suitable materials, e.g., polymers, metals or ceramics, in contrast to subtractive and formative methods of manufacturing. The three-dimensional object is built from a computer-aided design (CAD) model, usually by successively adding the materials in a layer-by-layer fashion.

The term "direct powder extrusion", DPE, relates to a 3D printing method involving the direct extrusion of a powder blend. The terms "direct powder extrusion printing" or "DPE printing" may be used synonymously. In case of a tablet, for example, DPE involves the direct extrusion of a powder blend, which may contain an API, through a nozzle, and the direct printing of a tablet by the deposition of the extruded blend according to the specified geometry. In contrast to FDM, for example, DPE is a one-step procedure omitting the step of preparing filaments using hot melt extrusion (see, for example, Goyanes et al. 2019; Sánchez-Guirales et al. 2021). The 3D printhead used in DPE comprises, for example, a single screw powder extruder fluidly connected to a nozzle, which can be moved in 3 dimensions.

The term "force-feeding" as used herein in relation to the manufacturing of a solid dosage form, e.g., a tablet, by additive manufacturing, in particular direct powder extrusion, relates to feeding a powder to, for example, a 3D printhead and/or an extruder being part of or upstream of a printhead, by mechanically assisting the flow of the powder, for example, by agitating the powder with a force feeder, e.g., a stirring element.

The term "mannitol" relates to Mannitol ($C_6H_{14}O_6$) in granular or powder form as a pharmaceutical excipient. Mannitol has three main polymorphic forms, $\alpha$, $\beta$, and $\delta$, the $\beta$ form being the thermodynamically most stable form under standard conditions. The term "compendial mannitol" refers to a mannitol conforming to quality standards specified in at least one pharmacopoeia, e.g., the European Pharmacopoeia (Ph. Eur.). The term "granular mannitol" relates to mannitol, e.g., D-mannitol, in granular form.

The term "sorbitol" relates to (2S,3R,4R,5R)-Hexane-1,2,3,4,5,6-hexol.

The term "copolymer of 1-vinyl-2-pyrrolidone and vinyl acetate" relates to "copovidone" (also "copolyvidone"; CAS 25086-89-9), a polyvinylpyrrolidone-vinyl acetate copolymer (PVP-VA), also referred to as "vinylpyrrolidone-vinyl acetate copolymer", a synthetic random copolymer of 1-ethenylpyrrolidin-2-one and ethenyl acetate in a ratio of 3:2 by mass, corresponding to the general chemical formula (I) below:

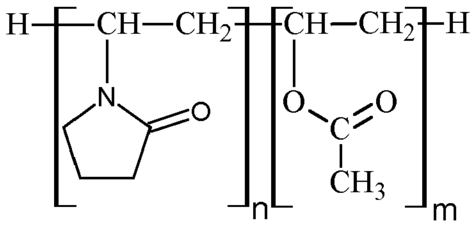

(I)

The ratio of n:m is ≈ 1.2:1.

The term "polyethylene glycol-polyvinyl alcohol graft copolymer", PEG-PVA, also referred to as "polyvinylalcohol-polyethylene glycol graft copolymer" or "macrogol-poly(vinyl alcohol) graft copolymer", (CAS 96734-39-3) relates to a synthetic grafted copolymer of, for example, approximately 25% polyethelene glycol (PEG) and approximately 75% polyvinyl alcohol (PVA) units with a molecular weight of approximately 40000-50000 Dalton, wherein the vinyl alcohol moieties are grafted on the polyethylene glycol backbone. Part of a PEG-PVA copolymer is shown in the general chemical formula (II) below:

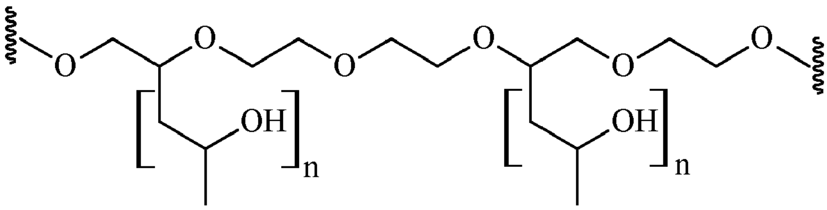

(II)

The term "pharmaceutically acceptable" in relation to a compound or composition, e.g., an excipient or excipient composition, means any compound or composition that is acceptable for veterinary use and in particular human use, i.e., is substantially safe and non-toxic for mammals, especially humans, and that has no substantial undesired effect on the biological activity of the active ingredient.

For the purposes of the present invention, the indication of a range such as "48-92", for example, is to be understood as meaning that each intermediate value is also disclosed. Any narrower range from a broader range is also meant to be disclosed by indicating the broader range, the narrower range also including ranges not comprising any or only one of the boundary values of the broader range, e.g., a range of 65-80 from a range of 48-92, or a range of 48-75 from a range of 48-92.

In a preferred embodiment, the excipient composition of the invention is composed of

a) 8-20 % by weight of the excipient composition of sugar alcohol, and
b) 80-92 % by weight of the excipient composition of a polymer blend, the polymer blend comprising, preferably being composed of, a first polymer component and a second polymer component in a weight ratio of 1:0.55 - 1:1.75, the first polymer component being a copolymer of 1-vinyl-2-pyrrolidone and vinyl acetate, and the second polymer component being polyethylene glycol-polyvinyl alcohol graft copolymer.

In a preferred embodiment of the excipient composition of the invention, the polymer blend comprises, preferably is composed of, the first polymer and the second polymer in a weight ratio of 1:0.65 - 1:1.7, preferably in a weight ratio of 1:0.75 - 1:1.7, further preferred in a weight ratio of 1:0.85 - 1:1.65, 1:0.9 -1:1.6, 1:0.95 - 1:1.5, 1:1.0 - 1:1.4, 1:1.05 - 1:1.3, 1:1.1 - 1:1.3, 1:1.15 - 1:1.25, or 1:1.15 - 1:1.2, most preferred in a weight ratio of 1.2.

In a preferred embodiment of the excipient composition of the invention, the sugar alcohol is selected from the group consisting of mannitol, sorbitol, xylitol, maltitol, lactitol, and erythritol, or a combination thereof. Preferably, the sugar alcohol is selected from mannitol or sorbitol, or a combination of mannitol and sorbitol. Further preferred, the sugar alcohol is mannitol, preferably granular mannitol.

In the excipient composition of the invention, the ratio (w/w) of the polymer blend to the sugar alcohol is preferably in the range of 4:1 - 11.5:1, more preferably 4.5:1 - 9.0:1 or 5:1 - 8.5:1.

In a second aspect, the invention relates to a pharmaceutical composition for the preparation of an amorphous solid dispersion or amorphous solid solution, the pharmaceutical composition comprising an excipient composition according to the first aspect of the invention, and an active pharmaceutical ingredient, API, or a composition of two or more active pharmaceutical ingredients, APIs.

The pharmaceutical composition according to the invention is particularly useful for the manufacturing of a pharmaceutical solid dosage form comprising an amorphous solid dispersion or amorphous solid solution, for example a solid oral dosage form or a powder, e.g., a high dose powder formulation. A solid oral dosage form, e.g., a tablet, can advantageously be manufactured by additive manufacturing, in particular by direct powder extrusion (DPE). A high dose powder formulation can, for example, be prepared by spray drying.

The pharmaceutical composition of the invention preferably comprises, or is composed of, in relation to the weight of the total pharmaceutical composition, >0-40 wt% of an active pharmaceutical ingredient, API, or a composition of two or more active pharmaceutical ingredients, and 60-<100 % by weight of the excipient composition according to the first aspect of the invention.

In preferred embodiments of the pharmaceutical composition according to the invention, the polymer blend of the excipient composition comprises, preferably is composed of, the first polymer and the second polymer in a weight ratio of 1:0.65 - 1:1.7, preferably in a weight ratio of 1:0.75 - 1:1.7, further preferred in a weight ratio of 1:0.85 - 1:1.65, 1:0.9 - 1:1.6, 1:0.95 - 1:1.5, 1:1.0 - 1:1.4, 1:1.05 - 1:1.3, 1:1.1 - 1:1.3, 1:1.15 - 1:1.25, or 1:1.15 - 1:1.2, most preferred in a weight ratio of 1.2.

In a preferred embodiment, the pharmaceutical composition of the invention comprises, preferably is composed of,

a) >0-40 wt%, based on the total weight of the pharmaceutical composition, of an active pharmaceutical ingredient, API, or a composition of two or more active pharmaceutical ingredients,

b) 8-12 wt%, based on the total weight of the pharmaceutical composition, of a sugar alcohol, and

c) 48-<92wt%, based on the total weight of the pharmaceutical composition, of the polymer blend described herein in relation to the first aspect of the invention, i.e., a polymer blend comprising, or consisting of, two polymers, namely a copolymer of 1-vinyl-2-pyrrolidone and vinyl acetate (copovidone, PVP-VA) and polyethylene glycol-polyvinyl alcohol graft copolymer (PEG-PVA). The polymers are present in the composition in a weight ratio of 1:0.55 - 1:1.75 (PVP-VA : PEG-PVA).

Suitable sugar alcohols are, for example, mannitol, sorbitol, xylitol, maltitol, lactitol, or erythritol. Preferred sugar alcohols are mannitol or sorbitol, or compositions of mannitol and sorbitol. Mannitol is preferably in a granular form. A suitable granular-grade of mannitol is, for example, commercially available under the name "Mannogem® XL Ruby" (SPI Pharma, USA). It has an average particle size of 300 μm. A suitable copovidone is, for example, commercially available under the name "Kollidon® VA 64 (KVA64; BASF). A suitable polyethylene glycol-polyvinyl alcohol graft copolymer, PEG-PVA, is, for example, commercially available under the name "Kollicoat® IR" (BASF).

In a preferred embodiment of the invention, the pharmaceutical composition comprises 10 % of mannitol, preferably granular mannitol, by weight of the total pharmaceutical composition.

In a particularly preferred embodiment, the pharmaceutical composition according to the invention comprises, or is composed of

a) 0.01-40 % by weight, based on the total pharmaceutical composition, of an active pharmaceutical ingredient, API, or a composition of two or more active pharmaceutical ingredients, APIs,

b) 8-12 %, preferably 10 %, by weight, based on the total pharmaceutical composition, of a sugar alcohol, preferably mannitol, more preferably granular mannitol, and

c) 48-91.99 % by weight, based on the total pharmaceutical composition, of a polymer blend as described herein.

In a particularly preferred embodiment, the pharmaceutical composition according to the invention comprises, or is composed of

a) 0.1-40 % by weight, based on the total pharmaceutical composition, of an active pharmaceutical ingredient, API, or a composition of two or more active pharmaceutical ingredients, APIs,

b) 8-12 %, preferably 10 %, by weight, based on the total pharmaceutical composition, of a sugar alcohol, preferably mannitol, more preferably granular mannitol, and

c) 48-91.9 % by weight, based on the total pharmaceutical composition, of a polymer blend as described herein.

In a further preferred embodiment, the pharmaceutical composition according to the invention comprises, preferably is composed of:

a) 0.5-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 48-91.5 % by weight, based on the total pharmaceutical composition, of a polymer blend as described herein.

In a further preferred embodiment, the pharmaceutical composition according to the invention comprises, preferably is composed of:

a) 1-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 48-91 % by weight, based on the total pharmaceutical composition, of a polymer blend as described herein.

In a further preferred embodiment, the pharmaceutical composition according to the invention comprises, preferably is composed of:

a) 5-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 48-87 % by weight, based on the total pharmaceutical composition, of a polymer blend as described herein.

In a further preferred embodiment, the pharmaceutical composition according to the invention comprises, preferably is composed of:

a) 10-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 48-82 % by weight, based on the total pharmaceutical composition, of a polymer blend as described herein.

In a further preferred embodiment, the pharmaceutical composition according to the invention comprises, preferably is composed of:

a) 20-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 48-72 % by weight, based on the total pharmaceutical composition, of polymer blend as described herein.

In a further preferred embodiment, the pharmaceutical composition according to the invention comprises, preferably is composed of:

a) 25-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 48-67 % by weight, based on the total pharmaceutical composition, of a polymer blend as described herein.

In a further preferred embodiment, the pharmaceutical composition according to the invention comprises, preferably is composed of:

a) 30-40 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and

c) 48-62 % by weight, based on the total pharmaceutical composition, of a polymer blend as described herein.

In a further preferred embodiment, the pharmaceutical composition according to the invention comprises, preferably is composed of:

a) 30-36 % by weight, based on the total pharmaceutical composition, of an active pharmaceutical ingredient, API, or a composition of two or more active pharmaceutical ingredients, APIs,
b) 8-12 %, preferably 10 %, by weight, based on the total pharmaceutical composition, of a sugar alcohol, preferably mannitol, more preferably granular mannitol, and
c) 52-62 % by weight, based on the total pharmaceutical composition, of a polymer blend as described herein.

In a further preferred embodiment, the pharmaceutical composition according to the invention comprises two or more active pharmaceutical ingredients. This embodiment is particularly useful for the preparation of a combination drug, combining two or more APIs in a single dosage form, e.g., a solid dosage form like a tablet. The two or more APIs can be included in the single dosage form in a customized amount and ratio, for example. This can help making it easier to take a medication composed of more than one active ingredient and/or improve adherence of a subject, for example.

In a further aspect, the invention relates to a solid dosage form comprising a pharmaceutical composition according to the invention. In a preferred embodiment, the solid dosage form is manufactured using a pharmaceutical composition according to the invention, by additive manufacturing or by spray drying, preferably by direct powder extrusion. In a preferred embodiment the solid dosage form is a tablet, further preferred an immediate release tablet, manufactured by additive manufacturing, preferably by direct powder extrusion.

In a further aspect the invention relates to a method of additive manufacturing of a pharmaceutical solid dosage form by direct powder extrusion, the method comprising feeding, heating, and extruding the pharmaceutical composition according to the invention.

In a preferred embodiment, the method of the invention comprises the step of force-feeding the pharmaceutical composition to an extruder. The force-feeding may, for example, be accomplished by mechanically agitating, e.g., stirring or vibrating, the pharmaceutical composition while feeding the composition to the extruder.

In the method of the invention, the printing temperature is preferably in the range of 140 - 220 °C, for example 180 °C. The temperature is chosen, inter alia, based on the temperature stability of the API(s) used in the composition and on the glass transition temperature and melting viscosity of the composition. The method is preferably practiced with a direct powder extrusion printer as described below. Further preferred, the method is for additive manufacturing, preferably direct powder extrusion printing, of a tablet, preferably an immediate release tablet.

In a further aspect, the invention relates to a direct powder extrusion printer, comprising an extruder being in fluid connection with a hopper for receiving a powder material to be fed into the extruder, the printer comprising a force feeder for force-feeding the powder material into the extruder. A "direct powder extrusion printer" (DPE printer) is understood to mean a 3D printer having a direct powder extrusion printhead, i.e., a printhead comprising an extruder, for example a single-screw extruder. The extruder is arranged, in relation to the direction of the powder flow, upstream from a nozzle through which the powder blend is extruded, and downstream from the hopper. The direction of the powder flow is preferably in the direction of gravity. The printhead is moveable in all three dimensions, preferably controlled by a computer being programmed to control the printhead based on 3D data.

In a still further aspect, the invention relates to the use of a pharmaceutical composition according to the invention for the preparation of a pharmaceutical solid dosage form, e.g., a tablet, for example an immediate release tablet, by additive manufacturing, preferably by direct powder extrusion. In a preferred embodiment, the pharmaceutical solid dosage form is prepared using a direct powder extrusion printer of the invention described above. The invention also relates to the use of a pharmaceutical composition according to the invention for the preparation of a pharmaceutical solid dosage form by spray drying. Such a solid dosage form preferably is a powder, further preferred a high dose powder.

The invention is explained in more detail below with reference to the accompanying figures and exemplary embodiments for illustrative purposes only.

BRIEF DESCRIPTION OF THE DRAWINGS

FIG. 1 schematically shows a sectional view of part of an embodiment of a direct powder printer according to the invention.

FIG. 2 DSC diagrams (only first heating cycle) of the APIs Levodopa (LD) and Carbidopa (CD); top - LD, bottom - CD.

FIG. 3: TGA thermogram of the APIs Levodopa (LD) and Carbidopa (CD).

FIG. 4: DSC Diagrams of the excipients (only first heating cycle), powder blend and filament; top to bottom: Kollicoat® IR, Kollidon® VA64, Mannitol, powder blend, filament.

FIG. 5: Isothermal section of DSC runs (exotherm up); top - CD, bottom - powder blend.

FIG. 6: Isothermal (180 °C) TGA thermogram of CD.

FIG. 7: Dissolution diagram (LD) of the six different tablets (n = 6).

FIG. 8: Dissolution diagram (CD) of the six different tablets (n = 6).

FIG. 1 shows, for an exemplary embodiment of a direct powder extrusion printer (DPE printer) according to the invention, a simplified sectional view through a DPE printhead 1. A motor 2 rotates an axis 5, and the axis 5 drives an extruder screw 11 of an extruder 7, which is configured as a single-screw extruder in the embodiment shown here. A hopper 6 for receiving a powder blend that is to be extruded is arranged upstream of the extruder, "upstream" in relation to the flow direction 11 of the powder blend. The extruder 7 is fluidly connected to a nozzle 9 through which the powder blend is extruded. The nozzle is arranged downstream of the extruder 7, in the direction 10 of the powder blend flow. A heating element 8 serves to heat the powder blend in the extruder 7. A holder 3 is mounted on the axis 5 upstream of the hopper 6 and rotates with the axis 5. A force-feeder 4, here configured as a stirring element, is fixedly connected to the holder 3, such that the force-feeder 4, i.e., the stirring element, is also rotated with the holder 3. The force-feeder 4 is arranged and dimensioned in such a way that it protrudes into the interior of the hopper 6 and thus into the powder blend located therein, so that the powder blend is agitated by the movement of the force-feeder 4.

Examples

For the following example, a mix of Levodopa (LD) and Carbidopa (CD) was chosen as model APIs. Aim was the development of a formulation with an excellent printability as well as a rapid disintegration, utilizing LD and CD as model APIs. This combination is very intriguing from the view of a hospital pharmacy, as inpatients with Parkinson's disease require up to seven doses per day in different dosages and possibly even release profiles that are often compiled from fragmented, marketed drug products. However, due to the symptoms (e.g., tremor and dysphagia) of the disease, the patients often have problems handling and swallowing (Buhmann et al. 2019) tablets. By utilizing 3DP to print the required dose in a singular personalized tablet (Dose and shape) therapy adherence could be improved. Further, this would also reduce the potential of medication errors, as it eliminates, for example, the need to compile (often done by caregivers) the correct dose with fragmented tablets.

1. Materials and Methods

1.1. Materials

LD and CD were gifted by Desitin Arzneimittel GmbH (Hamburg, Germany). Benserazide-HCl (BZ) was gifted by Deva Holding A.S. (Istanbul, Turkey). Mass values [mg] and dosages of tablets always refer to Carbidopa and Benserazide base, respectively. Kollidon® VA 64 (vinylpyrrolidone-vinyl acetate copolymer) and Kollicoat® IR (polyvinylalcohol-polyethylene glycol graft copolymer) were gifted by BASF SE (Ludwigshafen, Germany). Mannogem® XL Ruby (mannitol) and Compressol® SM (co-processed Sorbitol and Mannitol) were donated by SPI Pharma (Wilmington, NC, USA) via Lehmann&Voss&Co. KG (Hamburg, Germany). Sodium dihydrogen phosphate dihydrate ($NaH_2PO_4$, Emsure®), di-sodium hydrogen phosphate dihydrate ($Na_2HPO_4$, Emsure®), phosphoric acid ($H_3PO_4$, LiChropur™), hydrochloric acid (HCl, Titripur®) and water for HPLC analysis were purchased from Merck KGaA (Darmstadt, Germany). Methanol in gradient grade (Chemsolute®) was purchased from Th. Geyer GmbH & Co. KG (Renningen, Germany). FDS/Proud packaging material consisting of multiple layers (Polyethylene and Cellophane 300, 20 μm thickness each; Baxter code: P1601 [P2010]) was manufactured by Yuyuma Co, Ltd (Osaka, Japan). Brown glass packaging was purchased from Zscheile & Klinger GmbH (Hamburg, Germany).

1.2. Methods

1.2.1. Blending of powders

For each batch, 50 - 100 g of a blend of APIs and the excipients were weighed and blended with a FagronLab™ InvoMatic blender (Fagron GmbH & Co. KG, Glinde, Germany) at 50 rpm for 10 min. Table 1 shows the formulations that were evaluated.

**Table 1: Formulations evaluated. Percentages are weight percent.**

| Formulation | Levodopa [%] | Carbidopa [%] | Kollidon® VA64 [%] | Kollicoat® IR [%] | Plasticizer [%] |
|---|---|---|---|---|---|
| P20/34-M | 28 | 7.56 | 20 | 34.44 | 10 (Mannitol) |
| P25/29-M | 28 | 7.56 | 25 | 29.44 | 10 (Mannitol) |
| P30/24-M | 28 | 7.56 | 30 | 24.44 | 10 (Mannitol) |
| P25/29-CSM | 28 | 7.56 | 25 | 29.44 | 10 (Compressol® SM) |
| P25/29-CA | 28 | 7.56 | 25 | 29.44 | 10 (Citric acid) |

1.2.2. Flowability

Flowability of batch P25/29-M was determined in triplicate following Ph. Eur. 10.6/2.9.16 [28] on a PTG S3 powder analyzer (Pharma Test Apparatebau AG, Hainburg, Germany) using nozzle 1.

1.2.3. Angle of repose

Three measurements of the drained angle of repose method according to Ph. Eur. 10.6/2.9.36 (EDQM, Ph. Eur.) were conducted with batch P25/29-M on a PTG S3 powder analyzer (Pharma Test Apparatebau AG, Hainburg, Germany).

1.2.4. Bulk density and tapped density of powders

Bulk and tapped densities were measured using method 1 and batch P25/29-M as described in Ph.Eur. 10.6/2.9.34 (EDQM, Ph. Eur.). Tapped density was measured with an Engelsmann jolting volumeter type EU42E2/114S-WF (J. Engelsmann AG, Ludwigshafen, Germany). The measurements were performed three times. Hausner factor and Compressibility index were calculated as suggested (Carr 1965).

1.2.5. Particle size analysis by laser light diffraction

Laser diffraction measurements were performed with a LS 13 320 laser diffraction particle size analyzer equipped with a Tornado DPS module (Beckman Coulter Inc, Brea, CA, USA). Each API, excipient and the blend of batch P25/29-M were measured five times. Particle sizes were calculated using the Fraunhofer method.

1.2.6. Differential scanning calorimetry (DSC)

DSC analyzes were carried out with a DSC 1 device (Mettler-Toledo, LLC, Columbus, OH, USA) operated with STARe Software V16.10. Each single component of the formulation as well as the powder blend and the extruded filament (both P25/29-M) were analyzed. Three different methods were utilized. Nitrogen was used as a purging gas at a flow rate of 50 mL/min. Every sample was heated to 250 °C (10 °C/min) and kept at this temperature for 5 min. CD, LD, mannitol and the powder blend were also heated (10 °C/min) to and kept at 185 °C (10 min), then cooled to 25 °C (20 °C/min) and kept at this temperature for 5 min and reheated to 185 °C (10 °C/min) and kept at this temperature for 10 min. Ultimately, CD and the powder blend were also heated to 182 °C (10 °C/min) and kept at this temperature for 30 min.

1.2.7. Thermogravimetric analysis (TGA)

TGA analyzes were carried out with a Discovery TGA (TA Instruments, Waters, LLC, U.S.A.) and data was collected and analyzed with TA Instruments Trios software and percent mass loss or onset temperature were calculated.

Two different methods were utilized. Both APIs were subjected to a temperature ramp of 10 °C/min starting at 40 °C and concluding at 300 °C in open aluminum pans. CD was additionally subjected to an isothermal stress test at 180 °C for 30 min.

1.2.8. 3D printing of tablets (DPE)

The previously blended powders were 3D printed using a M3DIMAKER™ equipped with a DPE printhead coupled with a 0.4 mm nozzle (FabRx Ltd., London, United Kingdom). Computer-aided design (CAD) models of tablets were designed using Autodesk Tinkercad (Autodesk Inc., Mill Valley, CA, U.S.A.) and processed with Repetier-Host software (Hot-World GmbH & Co. KG, Willich, Germany). To enhance powder flow, the M3DIMAKER™ was modified with a stirring element 4 (see Fig. 1).

Tablets were printed using the following parameters: printing temperature: 180 °C; first layer height: 0.6 mm, layer height: 0.15-0.30 mm (adaptive slicing), infill: 60-100%, travel speed 70 mm/s, first layer speed 5 mm/s, perimeter speed 7.5-15 mm/s, infill speed: 15-20 mm/s with zero (Oblongs) or one (Cylinders and Donuts) top layer, two bottom layers and two shells.

The following dose range was identified as clinically desirable: 60-170/15-42.5 mg LD/CD. Each of the three shapes was printed in a small (60/15 mg) and large (170/42.5 mg) model. The CAD models had the following measures for the different dosages: small Donut: diameter 10.5 mm, height: 2.71-2.89 mm, inner hole diameter:4 mm; large Donut: diameter: 12 mm, height: 5.75 mm, inner hole diameter: 4 mm; small Cylinder: diameter 10.5 mm, height: 2.71 mm; large Cylinder: diameter 12 mm, height: 5.5-5.89 mm; small Oblong w/ reduced infill: 14x7x3.1 mm; large Oblong w/ reduced infill: 21x8.5x 5 mm.

1.2.9. Friability of uncoated tablets

Friability of the printed Donuts of batch P25/29-M was measured according to Ph. Eur. 10.6; 2.9.7 (EDQM, Ph. Eur.), using an AE-2 friability tester (Biomation GmbH, Jugenheim, Germany). 100 rotations were performed at 25 rpm. Afterwards, the tablets were dedusted, weighed and the loss of mass [%] was determined.

1.2.10. Resistance to crushing

The measurements were performed according to Ph. Eur. 10.6; 2.9.8 (EDQM, Ph. Eur.), using an Erweka TBH 525 TD hardness tester (Erweka GmbH, Langen, Germany). The Oblongs were aligned so that the force was applied longitudinal. If a measurement exceeded the maximum force of the device (500 N), a crushing strength of 500 N was assumed for calculations and comparisons. Ten measurements were recorded per batch.

1.2.11. Tensile strength

Tablet size measurements were conducted using a Digital ABS AOS Caliper (Mitutoyo Corporation, Kawasaki, Japan). Together with the previously described crushing strength values (1.2.10.) tensile strength ($\sigma$) of the printed tablets was calculated for the Cylinders (Equation 1) according to the following equation (1) as described by Fell et. al.1968:

$$\sigma = \frac{2P}{\pi DT} \qquad (1)$$

P = crushing strength [N]; D = tablet diameter [mm]; T = tablet thickness [mm]

For the calculation of the Donut's tensile strength, the inner hole of the tablets was subtracted from D. The Oblongs were calculated using their width as D.

1.2.12. Disintegration

Disintegration analyzes were conducted based on Ph. Eur. 10.6; 2.9.1 (EDQM, Ph. Eur.), with a ZT41 disintegration tester (Erweka GmbH, Langen, Germany). In order to keep consistent analytical conditions, the same medium (0.1 M HCl) as for dissolution experiments was used. Discs were used for disintegration testing.

To simplify the comparison of the different formulations shapes and sizes the average disintegration rate (Equation 2) was calculated.

$$\text{Average disintegration rate } \left[\frac{mg}{s}\right] = \frac{\text{Tablet mass [mg]}}{\text{Disintegration time [s]}} \qquad (2)$$

1.2.13. Dissolution and Content analysis

Dissolution experiments were carried out according to Ph. Eur. 10.6; 2.9.3 (EDQM, Ph. Eur.), with a Premiere 5100 Dissolution System (Distek Inc., North Brunswick Township, NJ, USA). 0.1 M HCl was used as dissolution medium as suggested in Ph.Eur. 10.6; 5.17.1 (EDQM, Ph. Eur.). The paddle speed was set to 100 rpm. Samples were taken after 5, 10, 15, 20, 30, 45, 60 and 90 min. A sinker was used for analyzes. For all small tablets and the large Oblongs a Distek "Japanese Basket" was used, whereas a custom 3D printed polylactic acid (PLA) sinker (not shown) was used for the large Cylinders and the large Donuts. If applicable, the dissolution profiles were compared using the f2 comparison (Moore and Flanner 1996).

The API content of the powder blend and the dissolution samples were analyzed and evaluated with a Nexera XR HPLC system and LabSolutions software version 6.83 (both Shimadzu Corp., Nakagyo-ku, Kyōto, Japan). The Nexera XR HPLC system consists of two degassing units (DGU-20A5R) and two HPLC-pumps (LC-20ADXR) as well as an autosampler (SIL-20ACXR) a column oven (FCV-34AH) and a communication bus module (CBM-20A). Samples were dissolved and diluted in 0.1 M HCl. Turbid samples were filtered through a 0.22 μm PTFE Filter. Prior to each analytical sequence, the column was equilibrated for 30 min and the autosampler purged for 5 min. HPLC samples were measured in duplicate with an injection volume of 20 μL. An endcapped RP-18 column (LiChrospher® 100 RP-18 endcapped (5 μm), LiChroCART® 250-4.6 HPLC Cartridge) coupled with a RP-18 guard column (LiChrospher® 100 RP-18 (5 μm), LiChroCART® 4-4 Guard Column) (both Merck KGaA, Darmstadt, Germany) was utilized during analysis. The column oven was set to 35 °C and the total flow rate was set to 1.25 mL/min. The mobile phase was a 90: 10 mixture of a 30 mM hydrogen phosphate buffer (20 mM $NaH_2PO_4$ + 10 mM $Na_2HPO_4$ at pH 4) and methanol. Both APIs were detected at a wavelength of 280 nm and showed retention times of 2.94 min (LD) and 4.30 min (CD). An external, six level calibration was used. LD was calibrated in the range of 25-250 μg/mL, whereas CD was calibrated in the range of 6.25-62.5 μg/mL.

For LD and BZ analytics a separate HPLC method was developed for the same equipment. The mobile phase was a 95:5 mixture of aforementioned hydrogen phosphate buffer (pH 2.5) and methanol. LD was detected at 280 nm and BZ at 268 nm. Retention times were 3.41 min (BZ) and 4.41 min (LD). The calibration for LD was the same as in the above-named method and BZ was calibrated in the same range as CD.

1.2.14. Stability analysis

Small Donuts of P25/29-M were packed in FDS/Proud packaging material Unit Dose Bags (UDB) using a FDS II Proud 336 device (Baxter International Inc., Deerfield, Il, USA) or brown glass stored in constant climate chambers (KBF P 240 and KBF S 240; Binder GmbH; Tuttlingen, Germany). The constant climate chambers were set to 25 °C / 60% RH and 40 °C / 75% RH as suggested for long term and accelerated stability testing (ICH Q1A (R2) 2003). Samples packed in UDB were stored for 7 days or 28 days in each of the previously mentioned conditions. Samples in brown glass were stored for 28 days in the 25 °C / 60% RH condition. After storage, the API contents of the samples were analyzed via HPLC as described in 2.2.6. Each sampling time comprised three samples.

1.2.15. Statistical analysis

IBM SPSS statistics Version 27.0.1.0 (IBM Corporation; Armonk, NY, USA) was used for statistical calculations. Results were compared with ANOVA. Significance was assumed for p-values < 0.05.

2. Results

It was possible to print LD and CD formulations in a hospital setting. The following subsections present detailed results regarding pre-manufacturing screening of the excipients and the powder blend. Moreover, the manufacturing process is illustrated and the resulting tablets are characterized thoroughly.

2.1. Powder screening

In order to ensure a constant feed rate, the flowability of the powder formulation is of high importance. Flowability has been reported to be a considerable challenge in DPE (Boniatti et al. 2021). The formulation P25/29-M showed

passable powder flow characteristics according to Carr's Classification (Carr 1965). Measured values of angle of repose (41.1° ± 0.12°), Hausner ratio (1.26 ± 0.02) and compressibility index (20.92% ± 1.08%) all characterize the blends flowability as passable. During the measurements, the powder flow hung up and had to be mechanically induced. Consequently, the flow time could not be measured. Median particle sizes of d50 = 15.3 μm (CD) up to d50 = 345 μm (mannitol) were measured, while the blend P25/29-M showed the following values: d10 = 19.2 μm, d50 = 96.1 μm, d90 = 258 μm. During printing, core flow behaviour of the powder resulted in insufficient and inconstant feeding of the DPE printhead by the screw. To eliminate this problem, the utilized DPE printhead was structurally modified. For this purpose, a wire was bent in to the shape of an open noose and fitted to the coupling element of the printhead. The wire functions as a force feeder (Fig. 1), by constantly moving the powder bed and thereby ensuring that the screw transports the powder blend to the heating zone and subsequently the nozzle of the DPE printhead. Through this modification, a constant powder feed rate was achieved, which was advantageous for printing tablets with uniform weight and API content.

Sound powder flow properties are a hallmark of high-quality pharmaceutical powders, which are intended to be used as an intermediate product for subsequent manufacturing processes like tabletting, capsule filling and 3DP. During DPE it is crucial to ensure a consistent powder flow, since the process parameters of the melt extrusion process and the movements of the DPE printhead need to be perfectly concerted in order to ensure a continuous process.

The powder blend P25/29-M showed passable flow properties in all conducted measurements. This is to be expected regarding the sizes of API particles (d50 = 51.8 μm (LD); d50 = 15.3 μm (CD)) and their large quantities of 28% w/w (LD) and 7.56% w/w (CD) within the blend. On the one hand, small particles show high cohesion, which worsens the powder flow (Abdullah & Geldart 1999). On the other hand, it is crucial to select small API particles to ensure a statistically equal distribution of the APIs in the powder blend. Additionally, it can be assumed, that the small particle size and large specific surface area of the APIs promotes a rapid and uniform dispersion or solution of the APIs in the polymer matrix. Moreover, bulk density of the powder blend is low (0.46 g/mL) which is associated with high friction in the powder bed and a deterioration of the powder flow. The powder hung up during the flow time measurements in the funnel of the powder analysis device, which resembles the shape of the powder reservoir of the utilized DPE printhead. This indicated that the powder flow during the printing process would probably be insufficient as well. It was observed, that the screw of the printhead created a cylindrical, core powder flow-resembling empty space between itself and the powder bed. This led to insufficient extrusion and ultimately resulted in the abortion of the printing processes. The addition of fine particulate silica as a glidant was tested. This, however, did not sufficiently enhance the powder flow. A constructed force feeder that was mounted onto the printhead was able to accomplish this task (Fig. 1), resulting in constant powder flow, a smooth uninterrupted printing process and autonomous build plate adhesion of the extruded mass. The process was also improved regarding the weight and content uniformity of the resulting tablets with this measure.

2.2. Thermal screening

As DPE is based on melt extrusion and thereby a thermally stressing process, it is essential to investigate which temperatures the APIs of interest tolerate. This, in turn, helps with the choice of suitable excipients and enables the selection of the printing parameters.

Literature states that LD has a melting temperature (Tm) of 284-285 °C (Ledeti et al. 2018) and CD melts at 207 °C (Ajit et al. 2018). Additionally, some sources (Carbidopa, www.chemspider.com/Chemical-Structure.31640) state that CD decomposes at the Tm. To confirm literature data, DSC (Fig. 2) and TGA (Fig. 3) experiments were carried out.

The DSC diagram of LD shows a glass transition at 179.6 °C followed by a molecular relaxation. As expected, no melting peak was found, as the analysis was stopped 34 °C below the literature melting point of 284 °C. The DSC diagram of CD shows a large endothermic event spanning from ca. 100 °C to about 165 °C and a second event slightly above 200 °C, which is, as described in the literature, followed by a decomposition. The thermogram of LD has no mass loss until about 275 °C, whereas the thermogram of CD shows a mass loss of about 8% between ca. 90-140 °C.

On the basis of the API data, the excipients were chosen. The excipients, as well as the powder blend (P25/29-M) and the resulting filament were subjected to the same DSC (Fig. 4) method as the APIs. According to literature, Kollicoat® IR has a glass transition temperature (Tg) of 45 °C and a Tm of 208 °C (Kolter et al. 2012). DSC showed a glass transition at about 48 °C and an endothermic event with an onset at 203 °C. Literature states that Kollidon® VA64 has a Tg of 101 °C (Kolter et al. 2012). The DSC diagram shows a glass transition at about 100 °C. Mannitol is a well characterized substance with a Tm of 165-170 °C (Jüptner 2022). DSC analysis revealed a large endothermic event with an onset of 165.9 °C.

After dissolution experiments CD (DSC and TGA) and the powder blend (DSC) were subjected to alternative thermoanalytical methods, which exposed the samples to an increased temperature (isothermal) for a prolonged time. The DSC diagram of CD (Fig. 5) shows, that after an exposition to 182 °C for 7-8 min CD begins to decompose, whereas no events can be found in the diagram of the powder blend (Fig. 5). The thermogram of CD (Fig. 6) shows a loss in mass of more than 20% over the first 10 min, which is followed by a decelerated mass loss until the end of the analysis.

The DSC (Fig. 2) and TGA (Fig. 3) results suggest that no problems were to be expected with LD during the

printing process, as no degradation was found up to 250 °C.

The large endothermic event observed for CD represents the release of the water of CD monohydrate. The molecular mass of anhydrous CD (226.23 g/mol) is 92.6% of the molecular weight of the monohydrate (244.24 g/mol), which corresponds well with the found reduction in mass to 92.55%. DSC confirmed the Tm. However, as CD clearly starts to decompose slightly before the Tm at about 195 °C (Fig. 3), the printing temperature had to be limited in order to establish a cushion. Therefore, the aim of the printing pre-experiments was to find an excipient combination, which enables a printing temperature of at least 15 °C below the onset of the decomposition (i.e., 180 °C).

DSC measurements (Fig. 4) of the polymers and mannitol confirmed literature data regarding Tg and Tm.

As expected, the glass transition of Kollidon® VA64 as well as the melting of Mannitol and Kollicoat® IR were observed in the DSC diagram of the optimized powder blend P25/29-M. The decomposition of CD was also found again, albeit at higher temperatures, whereas the release of the monohydrate is no longer visible. This was probably a result of the relatively small percentage of CD (7.56%) in the powder blend.

Lastly, the DSC diagram of the extruded filament showed some different results. There is a glass transition at about 80 °C; this reduction of Kollidon® VA64s Tg was probably result of the added mannitol, as the addition of plasticizers is known to reduce Tgs of polymers (Honary & Orafai 2002). Additionally, the melting peak of mannitol was not found. The plasticizer did probably not recrystallize due to the formation of a solid dispersion/solution after being melted together with the polymers. The endothermic event in the beginning of the decomposition could have been the beginning of the melting process of Kollicoat® IR, as the individual DSC of this polymer also showed a very broad melting peak, which is most probably due to the nature of polymers being blends of different chain lengths.

After dissolution experiments showed a loss in CD. Both CD as well as the powder blend were subjected to an alternative DSC method, to evaluate if the prolonged exposition to the printing temperature triggers decomposition.

Both DSC and TGA clearly showed that CD decomposes at distinctly lower temperatures as expected. It has to be noted that the thermogram (Fig. 6) starts at about 95% mass, as the monohydrate of the CD evaporated during the initial heating phase, which is not pictured in the thermogram.

Interestingly, the isothermal DSC data of the powder blend suggests a protective effect on the CD, as no decomposition or other events were visible over the course of 30 min.

2.3. Manufacturing process and formulation screening

Prior to DPE printing, the API contents of the powder blend P25/29-M were analyzed via HPLC which confirmed the uniformity of the blend (see chapter 2.6.).

Consistent printability, appreciable hardness as well as an immediate release profile and a precise API content were the most Critical Quality Attributes (CQA) of the Quality Target Product Profile (QTTP) of this tablet, that was intended for clinical use (Yu et al. 2014). Printing temperature, feed rate and infill pattern were identified as the most Critical Process Parameters (CPPs). Solubility of the formulation, decomposition temperature of the APIs and excipients as well as flowability of the powder blend were identified as the most Critical Material Attributes (CMAs).

The constitution of the formulation was constantly optimized. The percentage of the BCS class I APIs was gradually increased from 25-35.56%. Kollidon® VA 64 and Kollicoat® IR were selected as polymer components providing adequate water solubility. Mannitol, Compressol® SM or citric acid monohydrate were added as a plasticizer.

Initially the plasticizers were optimized (Table 1). Afterwards, the polymer ratios were evaluated for their influence on the disintegration speed of the formulation and its build plate adhesion, before finally the impact of three different shapes (Cylinder, Donut, Oblong) on the disintegration and later dissolution behavior was investigated.

Experiments with the three plasticizers showed that Mannitol- and Compressol® SM-containing formulations were printable, while the citric acid-based formulations could not be extruded. P25/29-M was used to evaluate the influence of the different shapes.

The Cylinder was used as the initial shape in printing experiments. Afterwards, the Donut with higher SA/V ratio (surface area to Volume) was introduced (Windolf et al. 2021, Goyanes et al. 2015). Ultimately, it was considered promising to experiment with the infill. The reduction of the infill results in a drastically increased SA/V at the cost of the tablets being considerably larger. Rather than increasing the diameter of the Cylinder, it was chosen to increase the area by introducing the oblong shape, as this shape is somewhat capsule like and should therefore be easier to swallow than a large cylindric tablet (Buhmann et al. 2019).

HPLC analysis confirmed the uniformity of the powder blend, which is crucial in order to achieve tablets of uniform content and mass.

Regarding the DPE process, three of four CQAs were achieved within the development of the formulation and manufacturing process. Consistent printability of the formulations on a glass plate, without manual aid or adhesion enhancement, was achieved through careful factorial optimization of the development process and the composition of the formulation. Moreover, the printhead was modified with the stirring element to ensure a constant material feed rate in the printhead (Fig. 1). In order for the tablets to be useful in a clinical setting, it was seen as crucial to develop a

process, that does not require manual aid. This would occupy time of the qualified personnel of a hospital pharmacy. Especially printing of the first layer on glass surface without manual adhesion aid could only be achieved through careful optimization of the feed rate without overextrusion, which would probably have resulted in bulkier tablets and weight inconsistencies. Through constant optimization of the constitution of the formulation and the shape, an immediate release profile and an appreciable hardness were achieved as well. Although extensive effort was spent for the thermal screening, only LD was able to tolerate the printing temperature, which was identified as a CPP. CD degraded partially during the printing process. Since the printing temperature was constantly monitored as a Process Analytical Technology (PAT), variations of the printing temperature were excluded for the degradation, since the temperature of the heating element was very consistent. In consecutive experiments filaments were extruded at lower temperatures. Unfortunately, none of the formulations offered the possibility to decrease the extrusion temperature below 150 °C, where degradation of CD was still present. Besides reduced CD content, an unidentified degradation product was visible during HPLC analyses. Below 150 °C the viscosity of the formulation was too high for the printer to extrude. However, the dissolution data of LD shows, that the process was very precise regarding the LD content of the tablets. Moreover, the immediate release profile was clearly shown. Further analysis tools such as NIR or an in-process balance could also be desirable PAT tools for further developments.

Instead of DOEs an iterative, factorial approach was used to develop the process, since DPE allows extremely small batches below 10 g formulation and swift changes of the process parameters through the software.

Regarding the formulation, the API amount was increased up to 35.56%, since the BCS class I APIs promote a quick dissolution of the whole formulation. Moreover, doses as high as 170/42.5 mg LD/CD require a high percentage of API to limit the tablets size and ensure an appreciable swallowability. Mannitol as a plasticizer has been used in the concentration of 10% for FDM printing (Windolf et al. 2021). Several different polymers were screened during the study. Some of these are known to exhibit a retarding influence on the release of the API, for example hydroxypropyl cellulose (15-35% of the tablet) (Honary & Orafai 2002). Therefore, they were not viewed as suitable due to the requirement of an immediate release tablet, although some of these showed a very good printability. Subsequently, the water-soluble polymers Kollidon® VA 64 and Kollicoat® IR were selected and used in different ratios. Kollidon® VA 64 is a water soluble and brittle polymer with a low Tg [Solanki et al. 2018, Fuenmayor et al. 2018, Shi et al. 2021). Kollicoat® IR was included as a second polymer as a plasticizer with less brittle characteristics and a higher melt viscosity, that promotes adhesion on the built plate during extrusion (Kolter et al. 2012). Its acceptable water solubility is an important aspect for the formulation's immediate release characteristic. Moreover, Kollicoat® IRs flexible character improved the hardness characteristics of the tablet and reduces their friability. Only a combination of both polymers led to fulfilment of all these characteristics.

The ratios of the polymers were optimized to achieve reliable printability through an ideal melt viscosity, a quick disintegration and sufficient tablet hardness. The polymer ratio of P25/29-M proved to provide a quick tablet disintegration as well as the most reliable printing process. P25/29-M was preferred to P25/29-CSM, since the latter did not show a faster disintegration, while the included Compressol® SM (co-processed Sorbitol and Mannitol) might increase the formulations vulnerability towards humidity since sorbitol is more hygroscopic than mannitol [Rice et al. 2020, Chen et al. 2020]. P25/29-CA could not be extruded, since the viscosity of the sample was too high for the screw to extrude the formulation. The plasticizing effect of citric acid has been demonstrated for hot melt extrusion, although higher concentrations than 10% were found to show a greater effect (Schilling et al. 2007).

Over the course of disintegration experiments the tablets formed a sphere-like shape. This prolonged the disintegration time, as a sphere combines maximal volume with minimal surface. Consequently, the Donut shape was introduced. The hole in the middle of the tablet increases the SA/V ratio and prevents the mechanistic formation of a sphere. Finally, the Oblongs were printed and showed the most appreciable results in disintegration experiments. Resulting from their reduced infill, they offer the highest specific surface of all tablets.

2.4. Hardness

The tested tablets (all P25/29-M) showed average crushing strength values ranging from 62.0 N ± 18.3 N for the small Donuts up to 465.5 N ± 58.3 N for the large Cylinders. The true average value of the Cylinders might be considerably higher, since seven of the ten samples were not crushed by the maximum force of the measuring apparatus of 500 N and 500 N was used as a value for the samples to calculate the results. Nevertheless, the large Cylinders showed significantly ($p < 0.001$) higher values than each other batch. The small Cylinders showed high crushing strengths of 236.5 N ± 70.5 N, which were significantly higher than all Donuts and Oblongs. The large Donuts showed crushing strengths of 172.9 N ± 21.7 N, which were significantly higher than the small Oblongs (107.0 N ± 10.7 N) and the small Donuts. The large Oblongs (131.7 N ± 30.8 N) show significantly higher values than the small donuts while the small Oblongs show no significant differences to the two prior batches.

Tensile strength with partly modified calculations (section 2.2.11.), favored the cylindrical tablets as well (small: 5.48 N/mm2 ± 1.67 N/$mm^2$; large: 4.85 N/$mm^2$ ± 0.65 N/$mm^2$). Again, the value for the large Cylinders was restricted

by the measurement device and would normally be even higher. Tensile strength for the small Oblongs was 3.10 N/mm$^2$ $\pm$ 0.31 N/mm$^2$ and therefore significantly higher compared to the large Oblongs 1.85 N/mm$^2$ $\pm$ 0.47 N/mm$^2$, which show the lowest values. Both Donut formulations (small: 2.18 N/mm$^2$ $\pm$ 0.64 N/mm$^2$; large: 2.26 N/mm$^2$ $\pm$ 0.27 N/mm$^2$) show no significant differences to either of the Oblongs.

Tablets of both tested Donut batches showed very low friability. The small Donuts showed 0.025% while the large Donuts showed an even lower value of 0.017%.

The DPE printing process as well as the selected tablet excipients and especially their employed quantities differ largely from conventionally manufactured compacted tablets. Conventional tablets are compacted out of powder or granules with high compaction force, which creates solid bonds between the powder particles where brittle fragmentation, plastic and elastic deformation play a role (York 1992, Nordström et al. 2012, Humbert-Droz et al. 1983). DPE printed tablets gain their hardness through solidification of the melt-extruded mass without compaction. Overall, the tablets show very appealing hardness values. Especially the addition of the more flexible polymer Kollicoat® IR and the plasticizer mannitol should be responsible for the increase in hardness as well as the reduction of the formulation's brittleness, which leads to decreased friability (Gong & Sun 2015). Decreased friability is especially important for a tablet, that was developed to be used in a hospital setting, since the personnel is exposed to the tablet through manual handling without primary packaging during manufacturing, packaging and administration to the patient. As expected, the Cylinders show the highest resistance to crushing values. The Donuts and Oblongs show lower hardness values than the Cylinders which are nonetheless very satisfying. Since both forms lack some stability because of empty space within the shape through the hole or reduced infill, they brake at lower forces.

2.5. Disintegration

Disintegration times and rates (Table 2) of every printable formulation were evaluated. For each constitution and shape, two tablet sizes were analyzed, respectively. The investigated tablet sizes represent the upper and lower end of the selected dose range.

Statistical evaluation of the disintegration time shows four significantly different groups. The first group consists of the two Oblongs; the second group spans the remaining small tablets as well as the large Donut; the third group is the large Cylinder of powder blend P20/34-M and the final group consists of the remaining large cylinders.

Comparing the disintegration rates, three formulations stick out: the large Oblongs showed a significantly higher disintegration rate ($p < 0.001$) than every other formulation. Additionally, the large Donuts as well as the small Oblongs, which were statistically similar, stand out. The large Cylinders of P20/34-M were also similar to the small Oblongs, but showed a significantly lower disintegration rate than the large Donuts. The remaining formulations were largely comparable.

**Table 2: Disintegration data of the different constitutions and shapes (n = 6)**

| Formulation | Shape | Tablet weight ± SD [mg] | Disintegration time ± SD [mm: ss] | Disintegration rate ± SD [mg/s] |
|---|---|---|---|---|
| P20/34-M | Cylinder | 216.62 ± 6.81 | 22:43 ± 03:15 | 0.16 ± 0.02 |
| | | 610.07 ± 9.21 | 42:42 ± 09:06 | 0.25 ± 0.05 |
| P25/29-M | Cylinder | 216.78 ±4.90 | 21:25 ± 03:34 | 0.17 ± 0.04 |
| | | 603.50 ± 7.44 | 53:09 ± 04:34 | 0.19 ± 0.02 |
| | Donut | 209.93 ± 3.75 | 22:42 ± 01:18 | 0.15 ± 0.01 |
| | | 616.95 ± 16.69 | 29:16 ± 06:24 | 0.36 ± 0.07 |
| | Oblong | 227.08 ± 2.30 | 12:42 ± 01:47 | 0.30 ± 0.05 |
| | | 598.57 ± 6.85 | 11:29 ± 01:44 | 0.89 ± 0.15 |
| PR.1.23-M | Cylinder | 216.08 ± 4.96 | 27:58 ± 02:34 | 0.13 ± 0.01 |
| | | 588.67 ± 4.44 | 53:43 ± 06:35 | 0.19 ± 0.02 |
| P25/29-CSMCylinder | | 225.78 ± 7.99 | 22:04 ± 02:25 | 0.17 ± 0.02 |
| | | 620.58 ± 6.88 | 53:46 ± 01:43 | 0.19 ± 0.01 |

Due to its considerably lower time and material consumption than dissolution experiments, disintegration testing was used as a surrogate method for quickly screening the powder blends for their potential to produce immediate release tablets.

The formulations mainly consist of water-soluble polymers and, as a result of the manufacturing process, most likely exhibit a minimal porosity of the individual extrudate strands. Therefore, no disintegration typical for compacted

tablets was expected but more of an erosion process of the slightly swelling surface, comparable to lozenges. This expectation was met and led to the introduction of the disintegration rate, which enables a better comparison of the different formulations.

Every formulation had a mean disintegration time of 54 min or less, which, due to the eroding (dissolving) rather than disintegrating process, can be seen as an indication for their dissolution time.

Most of the tablets stuck to the respective discs, due to swelling of the surface. This reduced the surface area that is in contact with the disintegration medium and thereby most likely decelerated the process. Additionally, the discs have holes and the tablets did not adhere uniformly, which resulted in differing influences on the singular tablets and thereby increased the standard deviations of the respective mean disintegration times. The large Oblongs were the only tablets that did not stick to the discs, which means that the disintegration tests of these was influenced to a lesser degree. As disintegration testing was solely considered as a screening method, disintegration times were considered promising. The Ph. Eur. (Chapter 5.17.1) mentions the release of at least 80% of the API in 45 min or less as a general acceptance criterion for immediate release tablets (EDQM Ph. Eur. 2021). Considering the mean disintegration times (< 54 min), even if potentially negatively influenced, it seems likely that every screened formulation could yield an immediate release tablet.

The interchange of the plasticizer mannitol to Compressol® SM did not exhibit an influence on the disintegration rate. Therefore, P25/29-M was considered as the most auspicious powder blend, as it exhibited the best printability and every formulation showed promising disintegration times.

Hence, P25/29-M was selected as the powder blend which would be used to examine the influence of the tablet shape. As expected, the Donut resulted in an accelerated disintegration, which became especially obvious for the large tablets, as the disintegration rate was significantly higher than that of the large Cylinders of every other powder blend. When the Oblongs were examined, the acceleration was even more distinct. The large Oblongs disintegrated significantly faster than the small tablets of every other formulation, which is equally shown in their significantly higher disintegration rate.

These results show the immense influence that is exerted by the different tablet shapes. However, this had to be expected, as the Donuts and Oblongs have a higher SA/V ratio than the Cylinders. Ultimately, this strengthens the position of 3DP in tablet manufacturing, as the oblong shape with a reduced infill cannot be produced by classical tableting via compaction.

2.6. Dissolution and Content analysis

Dissolution times of all shapes of P25/29-M were evaluated. Each shape was investigated in a larger (LD/CD: 170 mg/42.5 mg) and smaller (LD/CD: 60 mg/15 mg) dose. Every tablet dissolved completely during the experiments. Additionally, the powder blend was analyzed for its API content.

Content analysis of the powder blend showed recoveries of 104.47% ± 0.43% (LD) and 100.53% ± 0.3% (CD).

The dissolution diagram of LD (Fig. 7) shows that the six shapes can be classified in two groups. The three small shapes as well as the large Oblongs exhibit a very rapid dissolution. The small Donuts (10 min: 88.01% ± 10.73%) and Oblongs (10 min: 87.53% ± 12.63%) as well as the large Oblongs (85.44% ± 13.49%) release more than 80% of their LD in less than 10 min, whereas the small Cylinders (10 min: 78.80% ± 8.47%; 15 min: 98.20% ± 5.52%) release 80% slightly slower. The large Cylinders (45 min: 85.68% ± 5.76%) and Donuts (45 min: 97.82% ± 5.83%) reach that threshold between 30 min and 45 min. In each case about 100% of the theoretical LD dose was recovered (100.12% ± 1.19% (large Cylinders) to 104.72% ± 2.71% (small Donuts)).

The diagram of CD (Fig. 8) shows that only 59.54% ± 3.16% to 69.36% ± 10.76% of the theoretical API dose were recovered from the tablets. The dissolution behavior regarding the CD, however, seems comparable to that of LD.

Following the dissolution experiments and the subsequently adapted thermoanalyses to further investigate the thermolability of CD, extrusion experiments with BZ were conducted. For these experiments, the CD (P25/29-M) was substituted with BZ. The formulation was extruded between 125-155 °C and the extrudate analyzed via HPLC. BZ recoveries were 29.52% (125 °C) to 24.61% (155 °C) compared to the powder blend.

Dissolution data shows that it is possible to manufacture immediate release tablets with DPE. Four of six investigated formulations released more than 85% of the LD in less than 15 min. This is particularly appealing, because a BCS-based biowaiver approach defines these values as a threshold at which an f2 comparison is unnecessary to demonstrate similarity between two products (ICH M9 2023). Still, although the dissolution was considerably slower, the other two formulations are to be considered immediate release formulations, as they released more than 80% of the LD in less than 45 min (EDQM Ph. Eur. 2021) (see section 3.5.).

Unfortunately, the printing process seemed to trigger the decomposition of CD, as only about 60-70% were recovered from the completely dissolved tablets, although LD was recovered completely. Therefore, it was found necessary to further evaluate the thermal stability of CD (Section 3.2.). Nonetheless, the dissolution behavior of the remaining CD seemed to be comparable to that of LD, which had to be expected as both substances are BCS class I drugs.

Ultimately, it was expected that the different shapes exhibit a considerable influence on the dissolution behavior, due to their different SA/V ratio and disintegration results and that the influence would be more apparent with larger tablets. These expectations were thoroughly confirmed, as the time that was needed to release 80% of the LD from the large Oblongs was about a quarter of the time that was needed for the large Cylinders.

Subsequent experiments with BZ showed that this alternative DOPA decarboxylase inhibitor is even more thermolabile than CD. More than 70% of the substance was lost during extrusion, compared to 30-40% of the CD that was lost at much higher extrusion temperatures.

2.7. Stability analysis

Stability experiments were conducted to evaluate the formulations stability in its designated primary packaging. After storage in constant climate chambers the API contents were determined (Table 3):

**Table 3: LD and CD contents (mean ± SD) of small Donuts of P25/29-M, measured after storage in different conditions in different packaging.**

| Storage condition and packaging [°C/ %RH; days] | Levodopa content [%] | Carbidopa content [%] |
|---|---|---|
| 25/60; 7 in UDB | 101.48 ± 1.23 | 61.31 ± 1.40 |
| 25/60; 28 in UDB | 103.26 ± 2.08 | 57.00 ± 5.12 |
| 25/60; 28 in brown glass | 103.65 ± 2.10 | 62.33 ± 1.82 |
| 40/75; 7 in UDB | 97.84 ± 0.39 | 52.87 ± 0.58 |
| 40/75; 28 in UDB | 101.95 ± 1.83 | 46.97 ± 2.02 |

Compared to the results of the small Donuts in the previous section that were tested without storage, only the 40/75; 7 in UDB sample showed a significantly (p =0.004) lower LD content. All other samples show no significant differences to the initial samples, including the 40/75; 28 samples.

CD content did not decrease significantly for 25/60; 28 in brown glass and 25/60; 7 in UDB, while 25/60; 28 in UDB, 40/75; 7 in UDB and 40/75; 28 in UDB showed significantly lower CD contents.

The stability test gathered valuable data regarding the formulation's vulnerability towards increased humidity and temperature.

LD content of 40/75; 7 in UDB decreased significantly. This is most likely caused by a slightly lower weight of the three samples (average weight 207.7 mg) compared to the other samples (average weight 216.9 mg). Moreover, the 40/75; 28 in UDB showed no significantly lower content, despite having been exposed to the most stressful conditions for the longest time. This leads to the conclusion, that LD should tolerate both conditions and packagings for at least 28 days. This is completely sufficient for the intended clinical supply with CLMM, where tablets would be printed daily according to the patient's needs.

CD showed a moderate stability if packed in brown glass and in the mildest storage condition 25/60; 7 in UDB. In 25/60; 28 in UDB and in both accelerated condition samples meaningful amounts of Carbidopa degraded during the study. It has been shown that carbidopa is vulnerable to degradation processes as oxidation and hydrolysis [Choudhary et al. 2018, Subramanian et al. 2020]. The manufacturer of the UDBs investigated their moisture permeability thoroughly (Akasaki 2016). According to these investigations 3-15 g/m3 permeates the UDBs at 20 °C / 60% RH and 30 °C / 90% RH in 24 h, respectively. This exposure, which should be lower or absent in the brown glass packaging, led to a significant decrease of CD content. However, the 25/60; 7 in UDB indicates, that CD is stable enough for the intended use in CLMM, since the tablets are prepared daily and storage is very short for personalized tablets. Moreover, the environment in the pharmacy has to be monitored and kept under 25 °C.

3. Conclusion

It was possible to formulate an outstanding tablet formulation for direct extrusion printing, regarding, for example, immediate release, storage stability and hardness characteristics, in a hospital setting. Thus, the implementation of DPE into the closed-loop medication management (CLMM, see Baehr & Melzer 2018) with LD as API, for example, is feasible. It was shown that DPE printing of LD and CD formulations in a hospital setting is feasible. Four of the five developed formulations were successfully printed. The formulation P25/29-CA (plasticizer: citric acid) could not be printed. However, CD did not tolerate the manufacturing process, despite careful thermal screening. This highlights the importance of CPPs and CMAs during the early development stages and the risk assessment. Nevertheless, other BCS class I APIs with sufficient thermal stability should be printable with the formulation of the invention.

References

Abdullah, E.C. and D. Geldart, The use of bulk density measurements as flowability indicators. Powder Technology, 1999. 102(2): p. 151-165.

Ajit V.K., Arun G.K., Piush K., Rakesh P., Formulation and Development of Floating Microspheres containing Levodopa and Carbidopa, Asian J. Pharm. Tech. 2018; 8 (4):200-202, doi: 10.5958/2231-5713.2018.00032.6.

Akasaki, N., Spec P160_P2010 Ref. No. YPFP20160616. 2016, Yuyama Co, Ltd. p. 2. Auriemma G, Tommasino C, Falcone G, Esposito T, Sardo C, Aquino RP. Additive Manufacturing Strategies for Personalized Drug Delivery Systems and Medical Devices: Fused Filament Fabrication and Semi Solid Extrusion, Molecules. 2022; 27(9):2784, doi: 10.3390/molecules27092784.

Baehr, M. and S. Melzer, Closed Loop Medication Management: Arzneimitteltherapiesicherheit im Krankenhaus, 1. Edition ed. 2018, Berlin, Germany: MWV Medizinisch Wissenschaftliche Verlagsgesellschaft. 256.

Bhujbal S.V., Mitra, B., Jain U., Gong Y., Agrawal A., Karki S., Taylor L.S., Kumar S., Zhou Q., Pharmaceutical amorphous solid dispersion: A review of manufacturing strategies, Acta Pharmaceutica Sinica B, 11(8), 2021, 2505-2536, doi: 10.1016/j.apsb.2021.05.014.

Buhmann C, Bihler M, Emich K, Hidding U, Pötter-Nerger M, Gerloff C, Niessen A, Flügel T, Koseki JC, Nienstedt JC, Pflug C. Pill swallowing in Parkinson's disease: A prospective study based on flexible endoscopic evaluation of swallowing. Parkinsonism Relat Disord. 2019 May; 62:51-56. doi: 10.1016/j.parkreldis.2019.02.002.

Boniatti J, Januskaite P, Fonseca LBd, Viçosa AL, Amendoeira FC, Tuleu C, Basit AW, Goyanes A, Ré M-I. Direct Powder Extrusion 3D Printing of Praziquantel to Overcome Neglected Disease Formulation Challenges in Paediatric Populations. Pharmaceutics, 2021, 13(8):1114, doi: 10.3390/pharmaceutics13081114.

Carbidopa, published under www.chemspider.com/Chemical-Structure.31640.html (accessed Dec 16, 2022).

Carr, R.L., Evaluating flow properties of solids. Chemical Engineering, 1965, 72(18): p. 163-168.

Chamberlain R, Windolf H, Geissler S, Quodbach J, Breitkreutz J. Precise Dosing of Pramipexole for Low-Dosed Filament Production by Hot Melt Extrusion Applying Various Feeding Methods. Pharmaceutics. 2022; 14(1):216, doi:/10.3390/pharmaceutics14010216 Chen M, Zhang W, Wu H, Guang C, Mu W. Mannitol: physiological functionalities, determination methods, biotechnological production, and applications. Appl Microbiol Biotechnol. 2020 Aug;104(16):6941-6951, doi: 10.1007/s00253-020-10757-y.

Choudhary, A.N.C., Ajay; Dutta, Kamlesh K, Forced degradation study and validation of a RP-HPLC method for simultaneous estimation for drug content and release of Levodopa, Carbidopa and Entacapone in combined dosage form. The Pharma Innovation Journal, 2018. 7(8): 109-122.

EDQM, European Pharmacopoeia. 10 ed. Vol. 6. 2021, Stuttgart, Germany: Deutscher Apotheker Verlag.

Fanous M., Gold S., Muller S., Hirsch S., Ogorka J., Imanidis G., Simplification of fused deposition modeling 3D-printing paradigm: Feasibility of 1-step direct powder printing for immediate release dosage form production, International Journal of Pharmaceutics 578, 2020, 119124, doi: 10.1016/j.ijpharm.2020.119124.

Fell, J.T. and J.M. Newton, The tensile strength of lactose tablets. Journal of Pharmacy and Pharmacology, 1968. 20(8): p. 657-659, doi: 10.1111/j.2042-7158.1968.tb09832.x. Fuenmayor E, Forde M, Healy AV, Devine DM, Lyons JG, McConville C, Major I. Material Considerations for Fused-Filament Fabrication of Solid Dosage Forms. Pharmaceutics. 2018; 10(2):44, doi: /10.3390/pharmaceutics10020044.

Gioumouxouzis, Christos I., Karavasili, Christina, Fatouros, Dimitrios G., Recent advances in pharmaceutical dosage forms and devices using additive manufacturing technologies.Drug Discovery Today, doi: 10.1016/j.drudis.2018.11.019.

Goyanes A., Buanz A.B.M., Hatton G.B., Gaisford S., Basit A.W., 3D printing of modified-release aminosalicylate (4-ASA and 5-ASA) tablets, European Journal of Pharmaceutics and Biopharmaceutics 89, 2015, 157-162, doi: 10.1016/j.ejpb.2014.12.003.

Goyanes A, Robles Martinez P, Buanz A, Basit AW, Gaisford S., Effect of geometry on drug release from 3D printed tablets. Int J Pharm. 2015 Oct 30;494(2):657-663. doi: 10.1016/j.ijpharm.2015.04.069.

Goyanes, A., Allahham, N., Trenfield, S. J., Stoyanov, E., Gaisford, S., & Basit, A. W. (2019). Direct powder extrusion 3D printing: Fabrication of drug products using a novel single-step process, International Journal of Pharmaceutics, 567, 118471, doi: 10.1016/j.ijpharm.2019.118471.

Gong, X. and C.C. Sun, A new tablet brittleness index, European Journal of Pharmaceutics and Biopharmaceutics, 2015. 93: p. 260-266, doi: 10.1016/j.ejpb.2015.04.007.

Honary, S. and H. Orafai, The Effect of Different Plasticizer Molecular Weights and Concentrations on Mechanical and Thermomechanical Properties of Free Films. Drug Development and Industrial Pharmacy, 2002. 28(6): p. 711-715.

Hong X, Han X, Li X, Li J, Wang Z, Zheng A. Binder Jet 3D Printing of Compound LEV-PN Dispersible Tablets: An

Innovative Approach for Fabricating Drug Systems with Multicompartmental Structures. Pharmaceutics. 2021 Oct 25;13(11):1780, doi: 10.3390/pharmaceutics13111780.

Humbert-Droz P., Gurry R., Mordier D., Doelker E., Densification behaviour of drugs presenting availability problems. International Journal of Pharmaceutical Technology and Product Manufacture, 1983. 4(2): p. 29-35.

ICH Topic Q1A (R2), Stability Testing of new Drug Substances and Products. [Guideline] 2003; Available from: www.ema.europa.eu/en/ich-q1a-r2-stability-testing-new-drug-substances-drug-products-scientific-guideline (accessed Jan 24, 2023).

ICH M9, guideline on biopharmaceutics classification system-based biowaivers. [Guideline] 2020; Available from: www.ema.europa.eu/en/ich-m9-biopharmaceutics-classification-system-based-biowaivers-scientific-guideline (accessed Jan 4, 2023).

Jüptner, A.C., Einfluss von Dosiersystemen auf sprühgetrocknete Pulver zur Applikation im Respirationstrakt, in Mathematics and Natural Sciences. 2022, Kiel University. p. 19.

Kolter, K., Karl, M., Gryczke, A., Hot-Melt Extrusion with BASF Pharma Polymers, Extrusion Compendium, 2nd Revised and Enlarged Edition, 2012, BASF SE. 201.

Kissi EO, Nilsson R, Nogueira LP, Larsson A, Tho I. Influence of Drug Load on the Printability and Solid-State Properties of 3D-Printed Naproxen-Based Amorphous Solid Dispersion. Molecules. 2021; 26(15):4492, doi: 10.3390/molecules26154492.

Ledeti, A., Olariu, T., Caunii, A., Vlase, G., Circioban, D., Baul , B., Ledeti, I., Vlase, T., Murariu M., Evaluation of thermal stability and kinetic of degradation for levodopa in non-isothermal conditions. J Therm Anal Calorim 131, 1881-1888 (2018), doi: 10.1007/s10973-017-6671-z.

Malebari AM, Kara A, Khayyat AN, Mohammad KA, Serrano DR. Development of Advanced 3D-Printed Solid Dosage Pediatric Formulations for HIV Treatment. Pharmaceuticals. 2022; 15(4):435, doi: 10.3390/ph15040435.

Mathew E, Pitzanti G, Larrañeta E, Lamprou DA. 3D Printing of Pharmaceuticals and Drug Delivery Devices. Pharmaceutics. 2020; 12(3):266, doi: 1 0.3390/pharmaceutics 12030266. Mendibil X, Tena G, Duque A, Uranga N, Campanero MÁ, Alonso J. Direct Powder Extrusion of Paracetamol Loaded Mixtures for 3D Printed Pharmaceutics for Personalized Medicine via Low Temperature Thermal Processing. Pharmaceutics. 2021; 13(6):907, doi: 1 0.3390/pharmaceutics 13060907.

Moore, J.W. and H.H. Flanner, Mathematical comparison of dissolution profiles. Pharmaceutical technology, 1996. 20(6): p. 64-74.

Nagapudi, K. & Jona, J., 2008, Amorphous Active Pharmaceutical Ingredients in Preclinical Studies: Preparation, Characterization, and Formulation, Current Bioactive Compounds, 400, 213-224, doi: 10.2174/157340708786847852.

Nordström J, Klevan I, Alderborn G., A protocol for the classification of powder compression characteristics. Eur J Pharm Biopharm. 2012 Jan;80(1):209-16, doi: 10.1016/j.ejpb.2011.09.006.

Pandi P, Bulusu R, Kommineni N, Khan W, Singh M. Amorphous solid dispersions: An update for preparation, characterization, mechanism on bioavailability, stability, regulatory considerations and marketed products, Int J Pharm. 2020 Aug 30; 586:119560, doi: 10.1 016/j .ijpharm.2020.119560.

Pitzanti G, Mathew E, Andrews GP, Jones DS, Lamprou DA. 3D Printing: an appealing technology for the manufacturing of solid oral dosage forms. J Pharm Pharmacol. 2022 Oct 10;74(10):1427-1449, doi: 10.1093/jpp/rgab136.

Rice T, Zannini E, K Arendt E, Coffey A., A review of polyols - biotechnological production, food applications, regulation, labeling and health effects. Crit Rev Food Sci Nutr. 2020;60(12):2034-2051, doi: 10.1080/10408398.2019.1625859.

S'ari, M., Blade, H., Cosgrove, S., Drummond-Brydson, R., Hondow, N., Hughes L.P., Brown, A., Characterization of Amorphous Solid Dispersions and Identification of Low Levels of Crystallinity by Transmission Electron Microscopy, Mol. Pharmaceutics 2021, 18, 5, 1905-1919, doi: 10.1021/acs.molpharmaceut.0c00918.

Sánchez-Guirales SA, Jurado N, Kara A, Lalatsa A, Serrano DR. Understanding Direct Powder Extrusion for Fabrication of 3D Printed Personalised Medicines: A Case Study for Nifedipine Minitablets. Pharmaceutics. 2021; 13(10):1583, doi: 10.3390/pharmaceutics13101583.

Seoane-Viaño I, Januskaite P, Alvarez-Lorenzo C, Basit AW, Goyanes A. Semi-solid extrusion 3D printing in drug delivery and biomedicine: Personalised solutions for healthcare challenges. J Control Release. 2021 Apr 10, 332:367-389, doi: 10.1016/j.jconrel.2021.02.027.

Schilling S.U., Shah, N.H., Malick A.W., Infeld M.H., McGinity JW., Citric acid as a solid-state plasticizer for Eudragit RS PO, Journal of Pharmacy and Pharmacology, Volume 59, Issue 11, November 2007, Pages 1493-1500, doi: 10.1211/jpp.59.11.0005.

Serrano DR, Kara A, Yuste I, Luciano FC, Ongoren B, Anaya BJ, Molina G, Diez L, Ramirez BI, Ramirez IO, Sánchez-Guirales SA, Fernández-García R, Bautista L, Ruiz HK, Lalatsa A. 3D Printing Technologies in Personalized Medicine, Nanomedicines, and Biopharmaceuticals. Pharmaceutics. 2023; 15(2):313, doi: 10.3390/pharmaceutics15020313.

Shi K, Salvage JP, Maniruzzaman M, Nokhodchi A. Role of release modifiers to modulate drug release from fused deposition modelling (FDM) 3D printed tablets. Int J Pharm. 2021 Mar 15;597:120315, doi: 10.1016/j.ijpharm.2021.120315.

Solanki NG, Tahsin M, Shah AV, Serajuddin ATM. Formulation of 3D Printed Tablet for Rapid Drug Release by Fused Deposition Modeling: Screening Polymers for Drug Release, Drug-Polymer Miscibility and Printability. J Pharm Sci. 2018 Jan;107(1):390-401, doi: 10.1016/j .xphs.2017.10.021.

Subramanian, V.B., Konduru N., Katari N.K., Dongala T., Gundla R., A simple high-performance liquid chromatography method development for Carbidopa and Levodopa impurities: Evaluation of risk assessment before method validation by Quality by Design approach. Separation Science Plus, 2020. 3(11-12): 530-539, doi: 10.1002/sscp.202000029. Tagami T, Ito E, Kida R, Hirose K, Noda T, Ozeki T. 3D printing of gummy drug formulations composed of gelatin and an HPMC-based hydrogel for pediatric use, Int J Pharm. 2021 Feb 1;594:120118, doi: 10.1016/j.ijpharm.2020.120118.

Varghese R., Salvi S., Sood P., Karsiya P., Kumar D., Recent advancements in additive manufacturing techniques employed in the pharmaceutical industry: A bird's eye view, Annals of 3D Printed Medicine 8, 2022, 100081, doi: 10.1016/j.stlm.2022.100081.

Verbeeck, R. K., & Musuamba, F. T., The revised EMA guideline for the investigation of bioequivalence for immediate release oral formulations with systemic action. Journal of Pharmacy & Pharmaceutical Sciences, 2012, 15(3), 376-388, doi: 10.18433/J3VC8J.

Windolf H, Chamberlain R, Quodbach J. Predicting Drug Release from 3D Printed Oral Medicines Based on the Surface Area to Volume Ratio of Tablet Geometry. Pharmaceutics. 2021; 13(9):1453, doi: 10.3390/pharmaceutics13091453.

Windolf H, Chamberlain R, Breitkreutz J, Quodbach J. 3D Printed Mini-Floating-Polypill for Parkinson's Disease: Combination of Levodopa, Benserazide, and Pramipexole in Various Dosing for Personalized Therapy. Pharmaceutics. 2022; 14(5):931, doi: 10.3390/pharmaceutics14050931.

Yan TT, Lv ZF, Tian P, Lin MM, Lin W, Huang SY, Chen YZ. Semi-solid extrusion 3D printing ODFs: an individual drug delivery system for small scale pharmacy, Drug Dev Ind Pharm. 2020 Apr; 46(4): 531-538, doi: 10.1080/03639045.2020.1734018.

York, P., Crystal engineering and particle design for the powder compaction process. Drug Development and Industrial Pharmacy, 1992. 18(6-7): p. 677-721.

Yu I, Chen RK. A Feasibility Study of an Extrusion-Based Fabrication Process for Personalized Drugs, Journal of Personalized Medicine, 2020, 10(1):16, doi: 10.3390/jpm10010016.

Yu LX, Amidon G, Khan MA, Hoag SW, Polli J, Raju GK, Woodcock J. Understanding pharmaceutical quality by design. AAPS J. 2014 Jul;16(4):771-83. doi: 10.1208/s12248-014-9598-3.

Zhang J, Vo AQ, Feng X, Bandari S, Repka MA. Pharmaceutical Additive Manufacturing: a Novel Tool for Complex and Personalized Drug Delivery Systems. AAPS PharmSciTech. 2018 Nov;19(8):3388-3402, doi: 10.1208/s12249-018-1097-x.

## Claims

1. An excipient composition for the preparation of an amorphous solid dispersion or amorphous solid solution, the excipient composition comprising:

   a) 8-20 % by weight of the excipient composition of sugar alcohol, and
   b) 80-92 % by weight of the excipient composition of a polymer blend, the polymer blend comprising a first polymer component and a second polymer component in a weight ratio of 1:0.55 - 1:1.75, the first polymer component being a copolymer of 1-vinyl-2-pyrrolidone and vinyl acetate, and the second polymer component being polyethylene glycol-polyvinyl alcohol graft copolymer.

2. The excipient composition according to claim 1, the polymer blend comprising the first polymer and the second polymer in a weight ratio of 1:0.65 - 1:1.7, preferably in a weight ratio of 1:0.75 - 1:1.7, further preferred in a weight ratio of 1:0.85 - 1:1.65, 1:0.9 -1:1.6, 1:0.95 - 1:1.5, 1:1.0 - 1:1.4, 1:1.05 - 1:1.3, 1:1.1 - 1:1.3, 1:1.15 - 1:1.25, or 1:1.15 - 1:1.2, most preferred in a weight ratio of 1.2.

3. The excipient composition according to claim 1 or 2, wherein the sugar alcohol is selected from the group consisting of mannitol, sorbitol, xylitol, maltitol, lactitol, and erythritol, or a combination thereof, preferably selected from the group consisting of mannitol and sorbitol, or a combination thereof, further preferred is mannitol, preferably granular mannitol.

**4.** A pharmaceutical composition for the preparation of an amorphous solid dispersion or amorphous solid solution, the pharmaceutical composition comprising an excipient composition according to one of claims 1 to 3, and an active pharmaceutical ingredient, API, or a composition of two or more active pharmaceutical ingredients, APIs.

**5.** The pharmaceutical composition according to claim 4, the pharmaceutical composition comprising:

a) >0-40 % by weight, based on the total pharmaceutical composition, of an active pharmaceutical ingredient, API, or a composition of two or more active pharmaceutical ingredients, APIs,
b) 60-<100 % by weight of the excipient composition according to one of claims 1 to 3.

**6.** The pharmaceutical composition according to one of claims 4 or 5, comprising:

a) >0-40 % by weight, preferably 0.01-40 % by weight, further preferred 0.1-40 % by weight, 0.5-40 % by weight, 1-40 % by weight, or 5-40 % by weight, based on the total pharmaceutical composition, of an active pharmaceutical ingredient, API, or a composition of two or more active pharmaceutical ingredients, APIs,
b) 8-12 % by weight, preferably 10% by weight, based on the total pharmaceutical composition, of sugar alcohol, and
c) 48-<92 % by weight, preferably 48-91.99 % by weight, further preferred 48-91.9 % by weight, 48-91.5 % by weight, 48-91 % by weight, or 48-87 % by weight, based on the total pharmaceutical composition, of the polymer blend.

**7.** The pharmaceutical composition according to one of claims 4 to 6, wherein the composition comprises two or more active pharmaceutical ingredients.

**8.** A solid dosage form, comprising a pharmaceutical composition according to one of claims 4 to7.

**9.** The solid dosage form according to claim 8, wherein the solid dosage form, preferably a tablet, more preferably an immediate release tablet, is manufactured using the pharmaceutical composition according to one of claims 4 to 7, by additive manufacturing, preferably by direct powder extrusion.

**10.** A method of additive manufacturing of a pharmaceutical solid dosage form by direct powder extrusion, the method comprising feeding, heating, and extruding the pharmaceutical composition according to one of claims 4 to 7.

**11.** The method of claim 10, comprising the step of force-feeding the pharmaceutical composition to an extruder.

**12.** The method of one of claims 10 or 11, wherein the pharmaceutical composition according to one of claims 4 to 7 is heated to a temperature of 140-220 °C.

**13.** A direct powder extrusion printer, comprising a printhead (1) comprising an extruder (7) being in fluid connection with a hopper (6) for receiving a powder material to be fed into the extruder (7), the printer comprising a force feeder (4) for force feeding the powder material into the extruder (7).

**14.** The direct powder extrusion printer according to claim 13, wherein the extruder (7) is a single-screw extruder.

**15.** Use of a pharmaceutical composition according to one of claims 4 to 7 for the preparation of a pharmaceutical solid dosage form by additive manufacturing, preferably direct powder extrusion, or by spray drying.

**16.** The use according to claim 15, wherein the pharmaceutical solid dosage form, preferably a tablet, more preferably an immediate release tablet, is prepared using a direct powder extrusion printer according to one of claims 13 or 14.

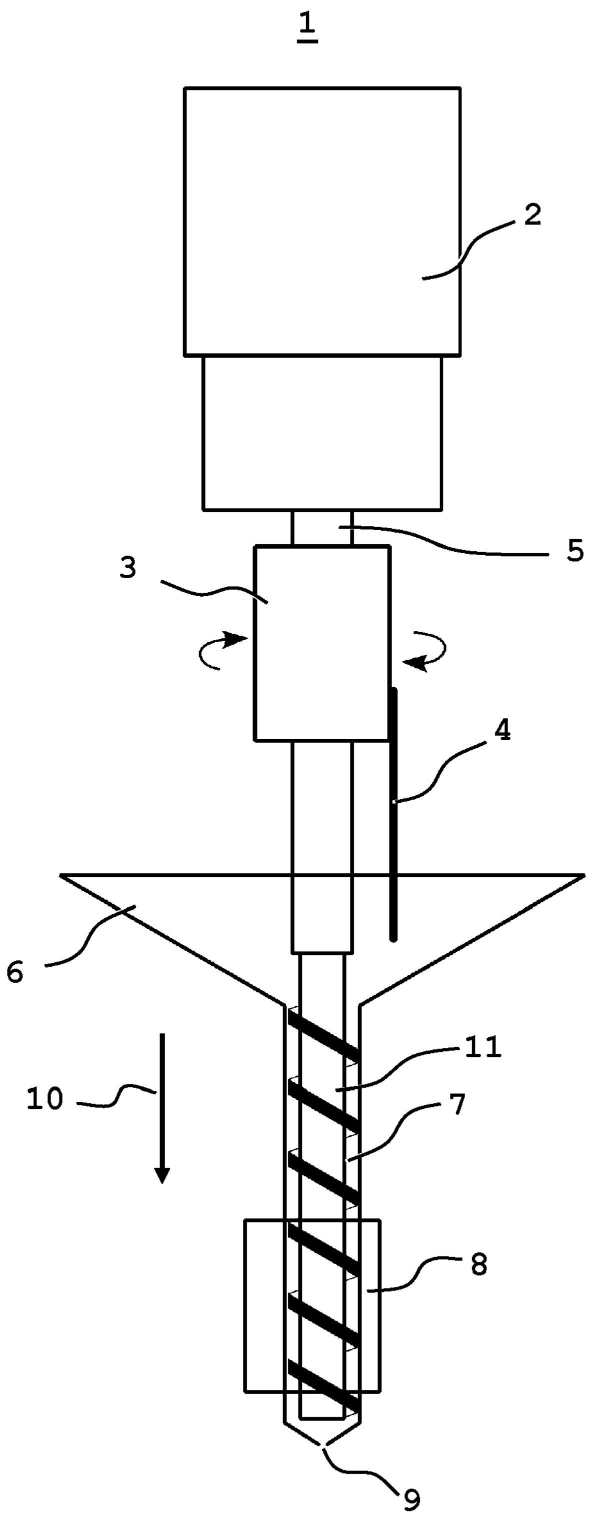


Fig. 1

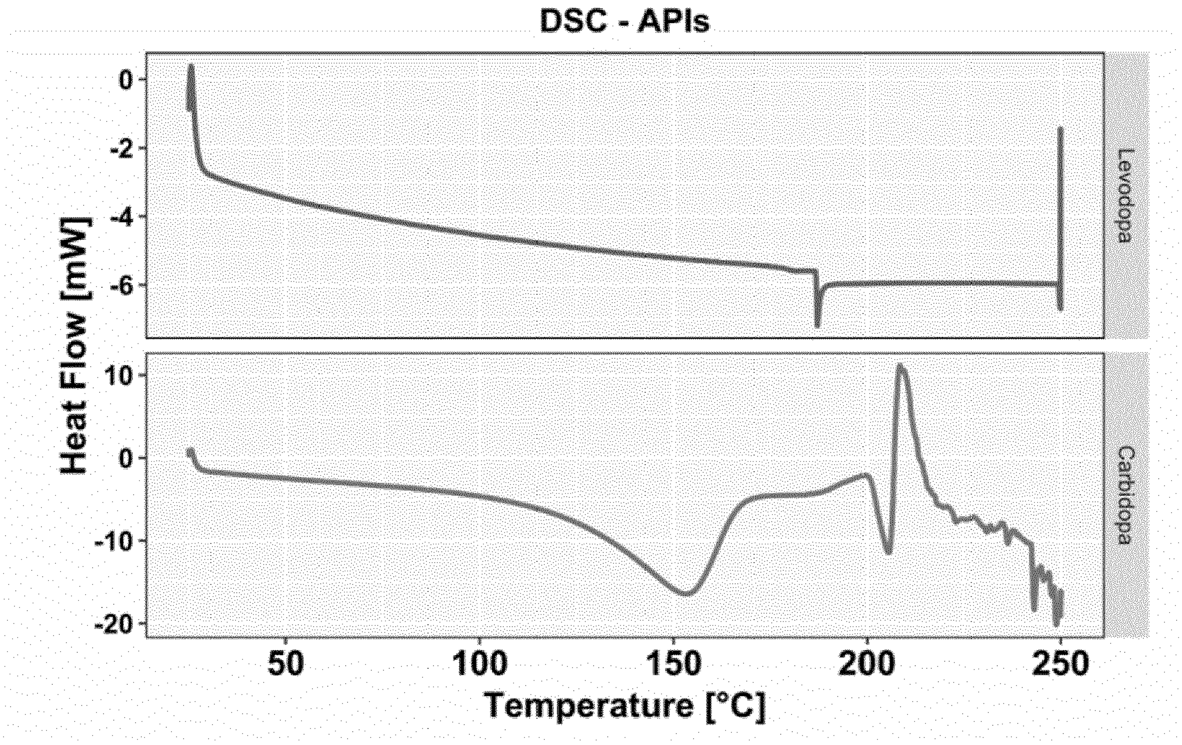
DSC - APIs
Heat Flow [mW]
0
-2
-4
-6
10
0
-10
-20
Levodopa
Carbidopa
50
100
150
200
250
Temperature [°C]

Fig. 2

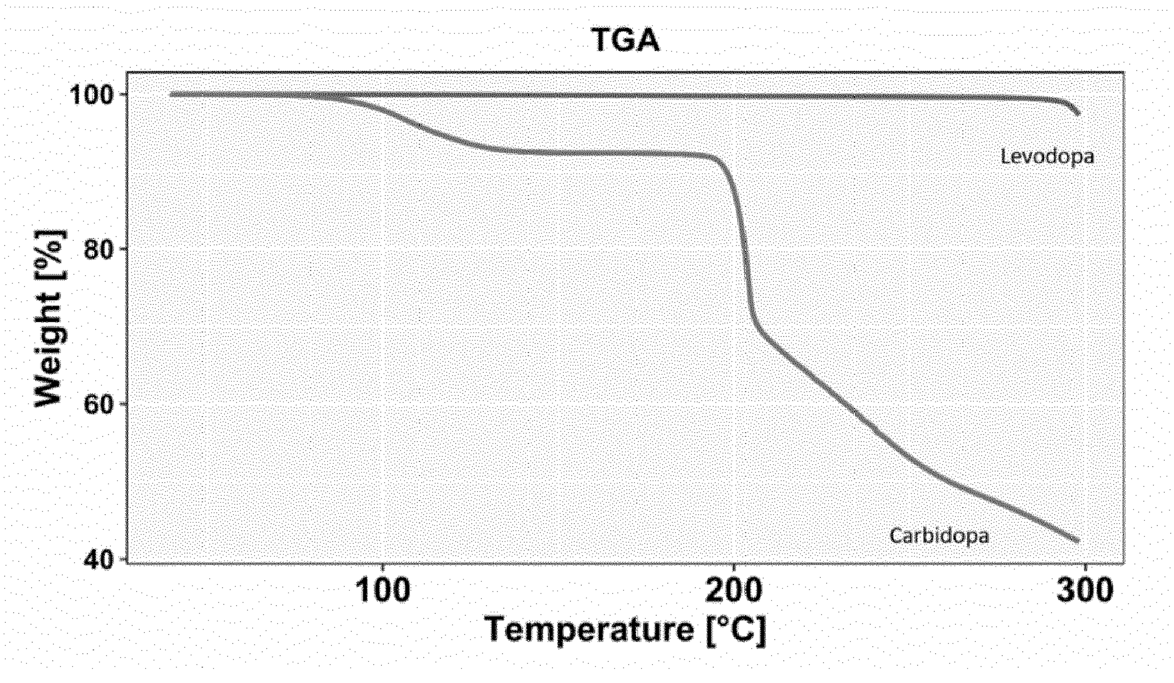
TGA
Weight [%]
100
80
60
40
Levodopa
Carbidopa
100
200
300
Temperature [°C]

Fig. 3

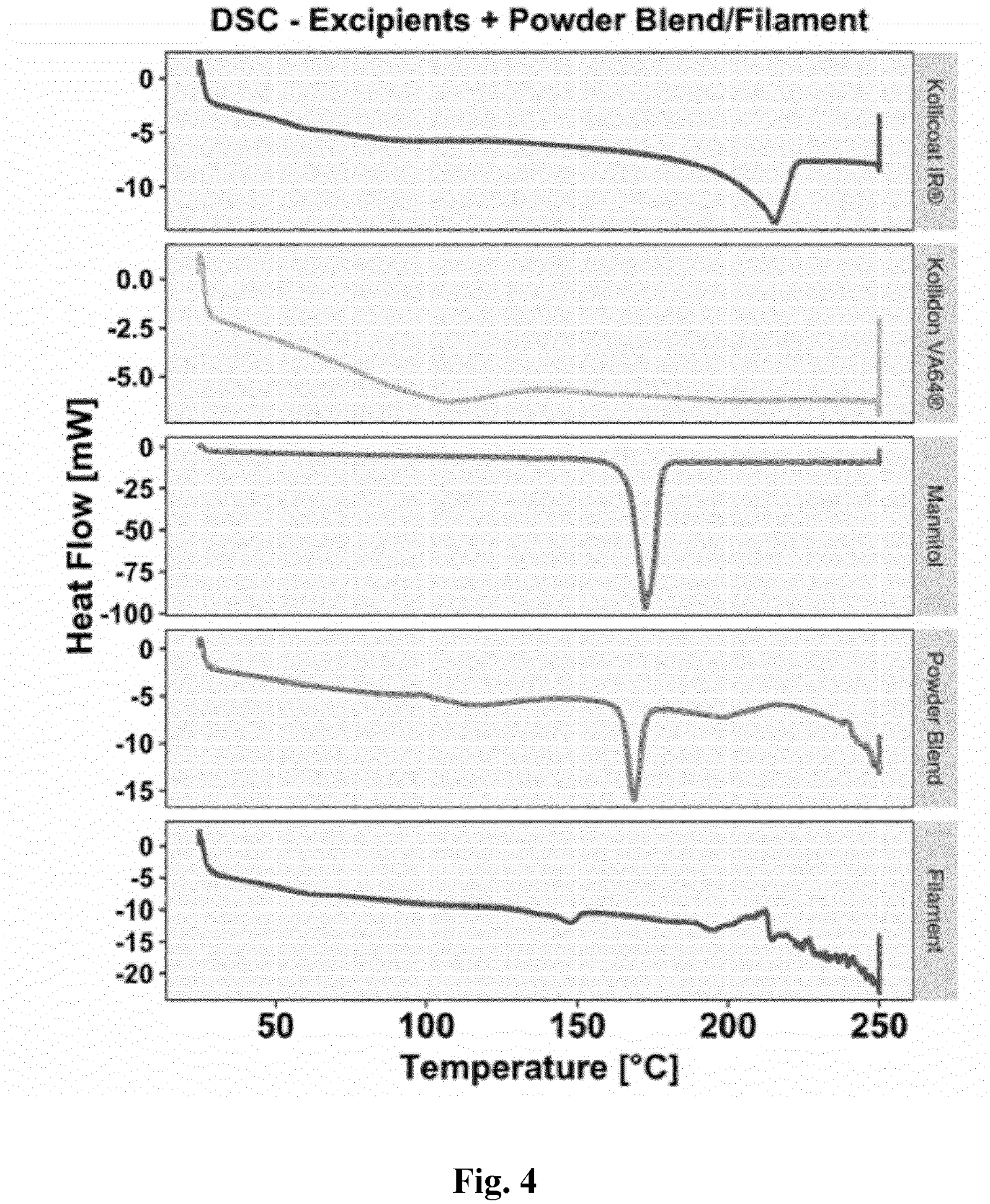
DSC - Excipients + Powder Blend/Filament
Heat Flow [mW]
Temperature [°C]
Kollicoat IR®
Kollidon VA64®
Mannitol
Powder Blend
Filament
0
-5
-10
0.0
-2.5
-5.0
0
-25
-50
-75
-100
0
-5
-10
-15
0
-5
-10
-15
-20
50
100
150
200
250

Fig. 4

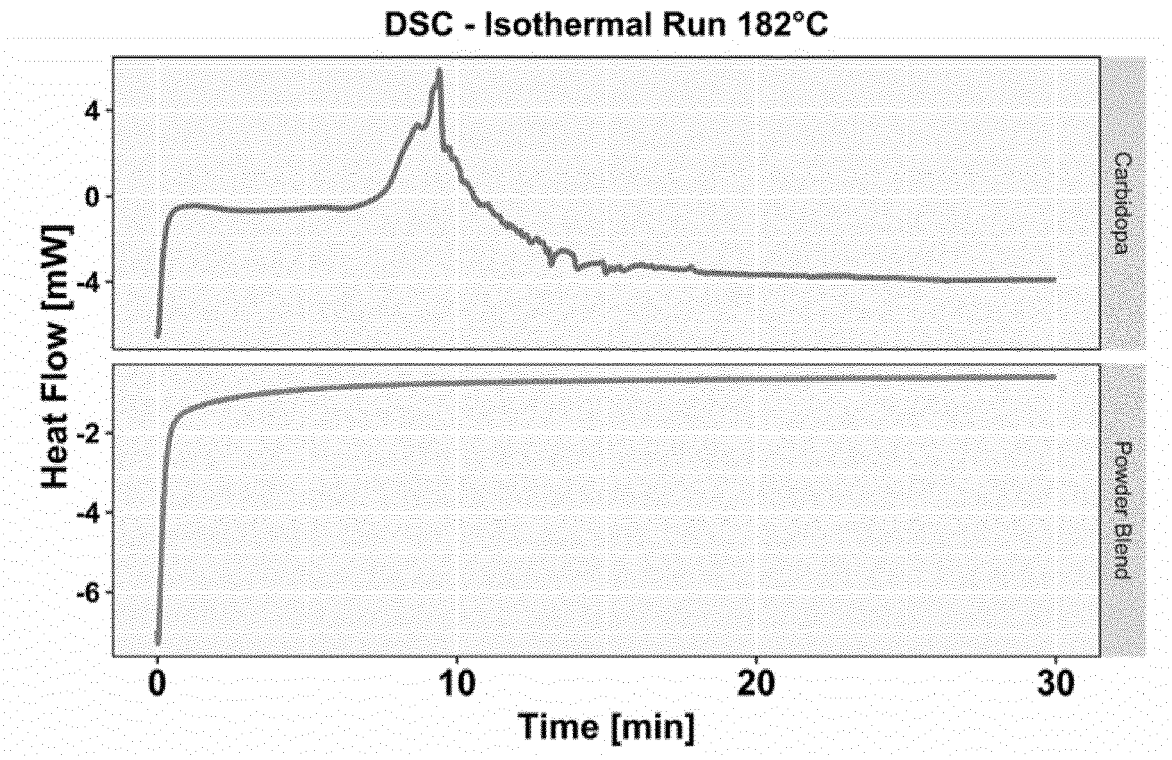
DSC - Isothermal Run 182°C
Heat Flow [mW]
4
0
-4
-2
-4
-6
Carbidopa
Powder Blend
0
10
20
30
Time [min]

Fig. 5

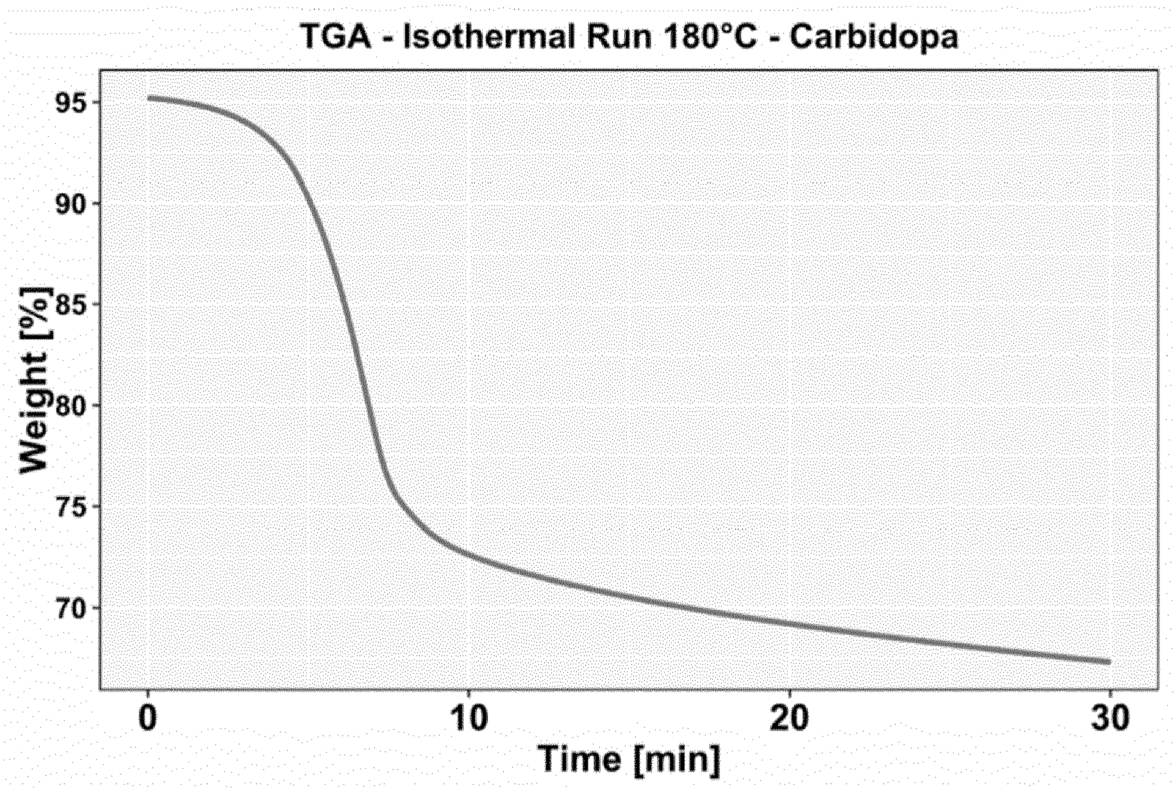
TGA - Isothermal Run 180°C - Carbidopa
Weight [%]
95
90
85
80
75
70
0
10
20
30
Time [min]

Fig. 6

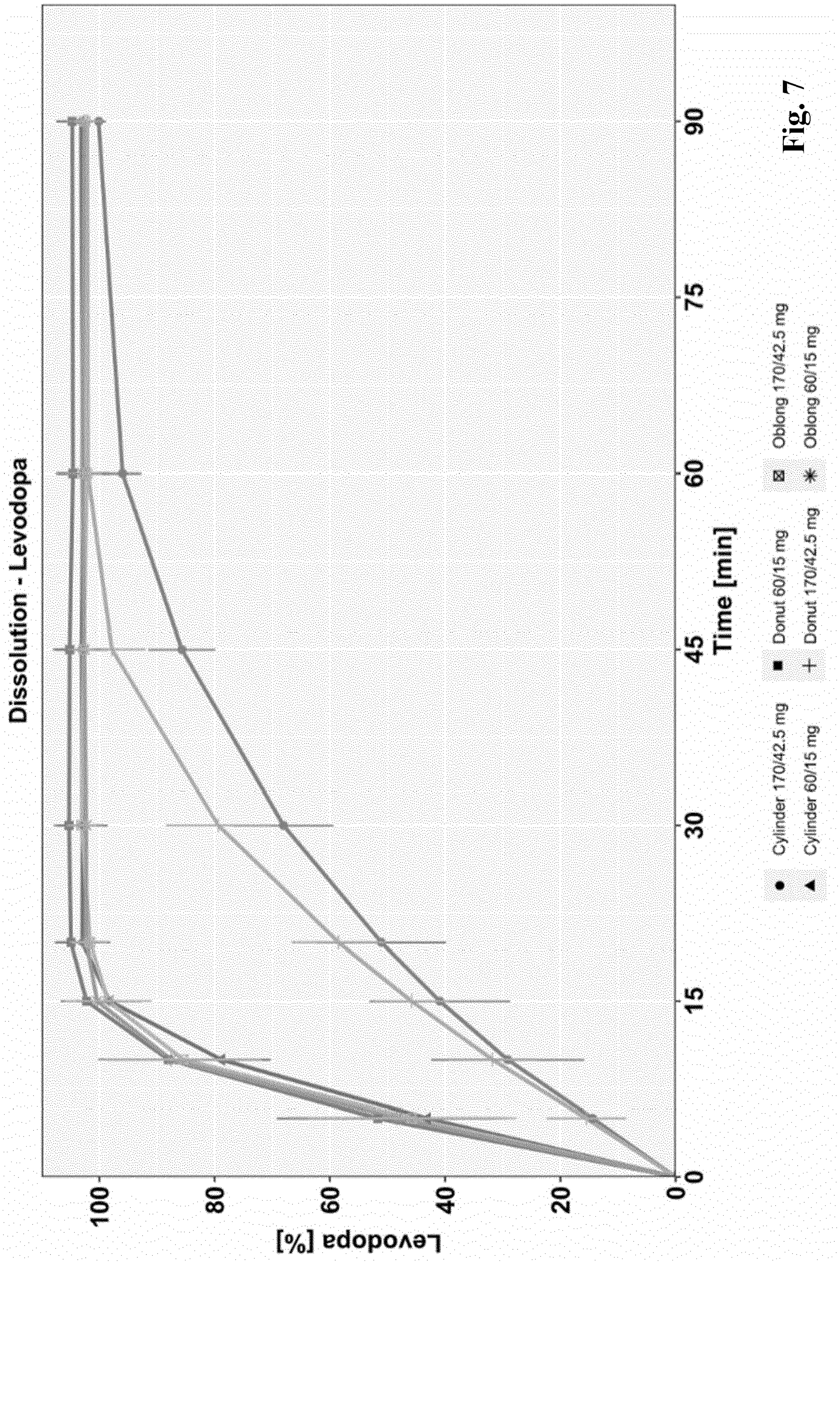
Dissolution - Levodopa
Levodopa [%]
0
20
40
60
80
100
Time [min]
0
15
30
45
60
75
90
Cylinder 170/42.5 mg
Cylinder 60/15 mg
Donut 60/15 mg
Donut 170/42.5 mg
Oblong 170/42.5 mg
Oblong 60/15 mg

Fig. 7

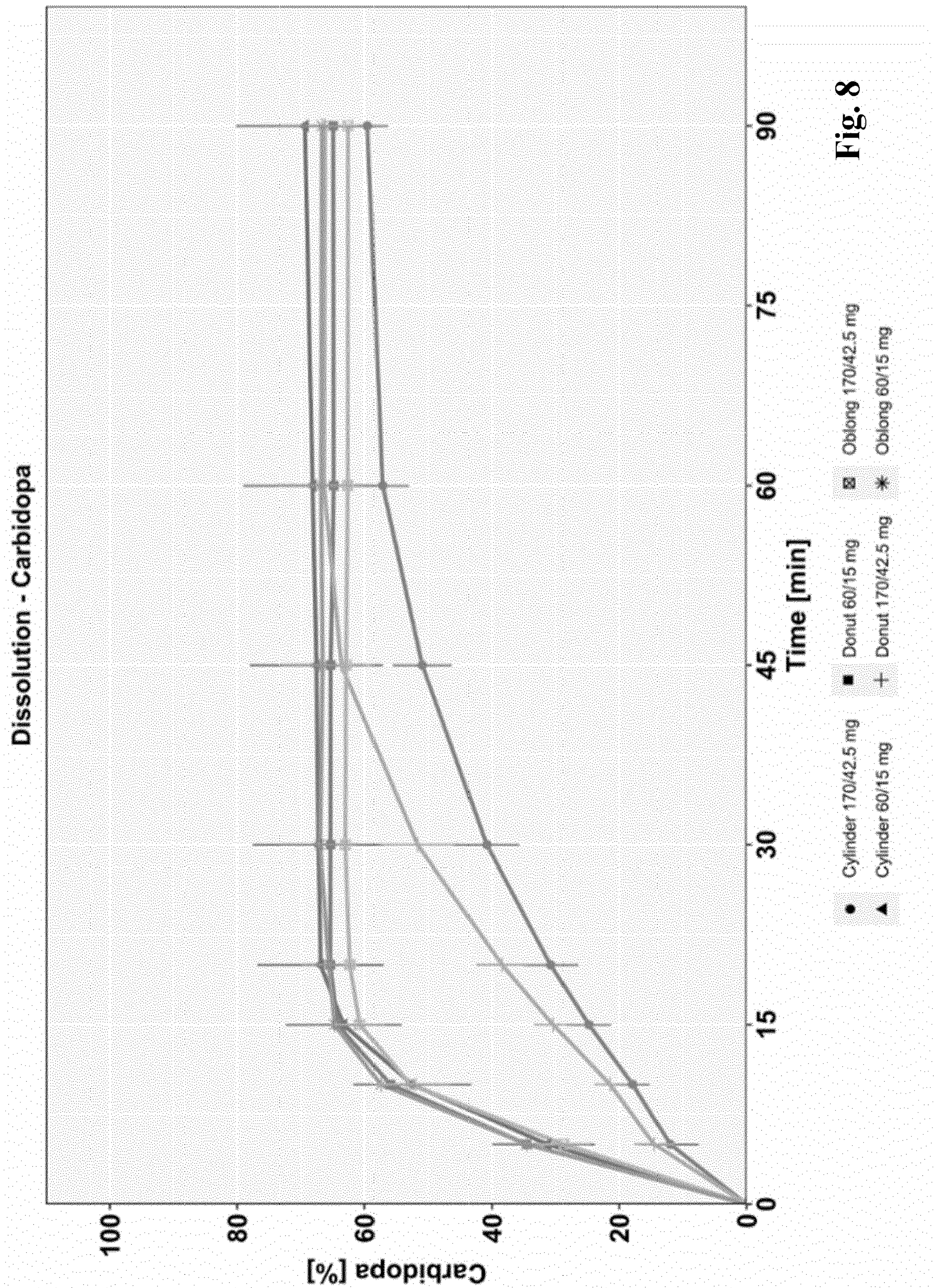
Dissolution - Carbidopa
Carbidopa [%]
0
20
40
60
80
100
0
15
30
45
60
75
90
Time [min]
Cylinder 170/42.5 mg
Cylinder 60/15 mg
Donut 60/15 mg
Donut 170/42.5 mg
Oblong 170/42.5 mg
Oblong 60/15 mg

Fig. 8

Europäisches Patentamt
European Patent Office
Office européen des brevets

# EUROPEAN SEARCH REPORT

Application Number
EP 23 15 6479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHUNG SOOYEON ET AL: "Development of ibuprofen tablet with polyethylene oxide using fused deposition modeling 3D-printing coupled with hot-melt extrusion", JOURNAL OF DRUG DELIVERY SCIENCE AND TECHNOLOGY, vol. 76, 1 October 2022 (2022-10-01), page 103716, XP93066173, FR ISSN: 1773-2247, DOI: 10.1016/j.jddst.2022.103716 | 13,14 | INV. A61K9/20 A61K31/00 A61K47/26 A61K47/32 A61P9/12 |
| A | * the whole document * | 1-12,15, 16 | |
| A | SHAMMA REHAB N ET AL: "Soluplus : A novel polymeric solubilizer for optimization of Carvedilol solid dispersions: Formulation design and effect of method of preparation", POWDER TECHNOLOGY, ELSEVIER, BASEL (CH), vol. 237, 29 December 2012 (2012-12-29), pages 406-414, XP028991212, ISSN: 0032-5910, DOI: 10.1016/J.POWTEC.2012.12.038 * the whole document * | 1-16 | |
| | | | TECHNICAL FIELDS SEARCHED (IPC) |
| | | | A61K A61P |

The present search report has been drawn up for all claims

1

| Place of search | Date of completion of the search | Examiner |
|---|---|---|
| Munich | 23 July 2023 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ABDULLAH, E.C. ; D. GELDART.** The use of bulk density measurements as flowability indicators. *Powder Technology,* 1999, vol. 102 (2), 151-165
- **AJIT V.K. ; ARUN G.K. ; PIUSH K. ; RAKESH P.** Formulation and Development of Floating Microspheres containing Levodopa and Carbidopa. *Asian J. Pharm. Tech.,* 2018, vol. 8 (4), 200-202
- **AKASAKI, N.** Spec P160_P2010 Ref. No. YPFP20160616. Yuyama Co, Ltd, 2016, 2
- **AURIEMMA G ; TOMMASINO C ; FALCONE G ; ESPOSITO T ; SARDO C ; AQUINO RP.** Additive Manufacturing Strategies for Personalized Drug Delivery Systems and Medical Devices: Fused Filament Fabrication and Semi Solid Extrusion. *Molecules,* 2022, vol. 27 (9), 2784
- **BAEHR, M. ; S. MELZER.** Closed Loop Medication Management: Arzneimitteltherapiesicherheit im Krankenhaus. MWV Medizinisch Wissenschaftliche Verlagsgesellschaft, 2018, 256
- **BHUJBAL S.V. ; MITRA, B. ; JAIN U. ; GONG Y. ; AGRAWAL A. ; KARKI S. ; TAYLOR L.S. ; KUMAR S. ; ZHOU Q.** Pharmaceutical amorphous solid dispersion: A review of manufacturing strategies. *Acta Pharmaceutica Sinica,* 2021, vol. 11 (8), 2505-2536
- **BUHMANN C ; BIHLER M ; EMICH K ; HIDDING U ; PÖTTER-NERGER M ; GERLOFF C ; NIESSEN A ; FLÜGEL T ; KOSEKI JC ; NIENSTEDT JC.** Pill swallowing in Parkinson's disease: A prospective study based on flexible endoscopic evaluation of swallowing. *Parkinsonism Relat Disord.,* May 2019, vol. 62, 51-56
- **BONIATTI J ; JANUSKAITE P ; FONSECA LBD ; VIÇOSA AL ; AMENDOEIRA FC ; TULEU C ; BASIT AW ; GOYANES A ; RÉ M-I.** Direct Powder Extrusion 3D Printing of Praziquantel to Overcome Neglected Disease Formulation Challenges in Paediatric Populations. *Pharmaceutics,* 2021, vol. 13 (8), 1114
- *Carbidopa,* 16 December 2022, www.chemspider.com/Chemical-Structure.31640.html
- **CARR, R.L.** Evaluating flow properties of solids. *Chemical Engineering,* 1965, vol. 72 (18), 163-168
- **CHAMBERLAIN R ; WINDOLF H ; GEISSLER S ; QUODBACH J ; BREITKREUTZ J.** Precise Dosing of Pramipexole for Low-Dosed Filament Production by Hot Melt Extrusion Applying Various Feeding Methods. *Pharmaceutics,* 2022, vol. 14 (1), 216
- **CHEN M ; ZHANG W ; WU H ; GUANG C ; MU W.** Mannitol: physiological functionalities, determination methods, biotechnological production, and applications. *Appl Microbiol Biotechnol.,* August 2020, vol. 104 (16), 6941-6951
- **CHOUDHARY, A.N.C. ; AJAY; DUTTA ; KAMLESH K.** Forced degradation study and validation of a RP-HPLC method for simultaneous estimation for drug content and release of Levodopa, Carbidopa and Entacapone in combined dosage form. *The Pharma Innovation Journal,* 2018, vol. 7 (8), 109-122
- EDQM, European Pharmacopoeia. Deutscher Apotheker Verlag, 2021, vol. 6
- **FANOUS M. ; GOLD S. ; MULLER S. ; HIRSCH S. ; OGORKA J. ; IMANIDIS G.** Simplification of fused deposition modeling 3D-printing paradigm: Feasibility of 1-step direct powder printing for immediate release dosage form production. *International Journal of Pharmaceutics,* 2020, vol. 578, 119124
- **FELL, J.T. ; J.M. NEWTON.** The tensile strength of lactose tablets. *Journal of Pharmacy and Pharmacology,* 1968, vol. 20 (8), 657-659
- **FUENMAYOR E ; FORDE M ; HEALY AV ; DEVINE DM ; LYONS JG ; MCCONVILLE C ; MAJOR I.** Material Considerations for Fused-Filament Fabrication of Solid Dosage Forms. *Pharmaceutics,* 2018, vol. 10 (2), 44
- **GIOUMOUXOUZIS, CHRISTOS I. ; KARAVASILI, CHRISTINA ; FATOUROS, DIMITRIOS G.** Recent advances in pharmaceutical dosage forms and devices using additive manufacturing technologies. *Drug Discovery Today*
- **GOYANES A. ; BUANZ A.B.M. ; HATTON G.B. ; GAISFORD S. ; BASIT A.W.** 3D printing of modified-release aminosalicylate (4-ASA and 5-ASA) tablets. *European Journal of Pharmaceutics and Biopharmaceutics,* 2015, vol. 89, 157-162
- **GOYANES A ; ROBLES MARTINEZ P ; BUANZ A ; BASIT AW ; GAISFORD S.** Effect of geometry on drug release from 3D printed tablets. *Int J Pharm.,* 30 October 2015, vol. 494 (2), 657-663

- **GOYANES, A. ; ALLAHHAM, N. ; TRENFIELD, S. J. ; STOYANOV, E. ; GAISFORD, S. ; BASIT, A. W.** Direct powder extrusion 3D printing: Fabrication of drug products using a novel single-step process. *International Journal of Pharmaceutics,* 2019, vol. 567, 118471
- **GONG, X. ; C.C. SUN.** A new tablet brittleness index. *European Journal of Pharmaceutics and Biopharmaceutics,* 2015, vol. 93, 260-266
- **HONARY, S. ; H. ORAFAI.** The Effect of Different Plasticizer Molecular Weights and Concentrations on Mechanical and Thermomechanical Properties of Free Films. *Drug Development and Industrial Pharmacy,* 2002, vol. 28 (6), 711-715
- **HONG X ; HAN X ; LI X ; LI J ; WANG Z ; ZHENG A.** Binder Jet 3D Printing of Compound LEV-PN Dispersible Tablets: An Innovative Approach for Fabricating Drug Systems with Multicompartmental Structures. *Pharmaceutics,* 25 October 2021, vol. 13 (11), 1780
- **HUMBERT-DROZ P. ; GURRY R. ; MORDIER D. ; DOELKER E.** Densification behaviour of drugs presenting availability problems. *International Journal of Pharmaceutical Technology and Product Manufacture,* 1983, vol. 4 (2), 29-35
- *ICH Topic Q1A (R2), Stability Testing of new Drug Substances and Products,* 2003, www.ema.europa.eu/en/ich-q1a-r2-stability-testing-new-drug-substances-drug-products-scientific-guideline
- *ICH M9, guideline on biopharmaceutics classification system-based biowaivers,* 2020, www.ema.europa.eu/en/ich-m9-biopharmaceutics-classification-system-based-biowaivers-scientific-guideline
- Einfluss von Dosiersystemen auf sprühgetrocknete Pulver zur Applikation im Respirationstrakt. **JÜPTNER, A.C.** Mathematics and Natural Sciences. Kiel University, 2022, 19
- Hot-Melt Extrusion with BASF Pharma Polymers. **KOLTER, K. ; KARL, M. ; GRYCZKE, A.** Extrusion Compendium. BASF, 2012
- **KISSI EO ; NILSSON R ; NOGUEIRA LP ; LARSSON A ; THO I.** Influence of Drug Load on the Printability and Solid-State Properties of 3D-Printed Naproxen-Based Amorphous Solid Dispersion. *Molecules,* 2021, vol. 26 (15), 4492
- **LEDETI, A. ; OLARIU, T. ; CAUNII, A. ; VLASE, G. ; CIRCIOBAN, D. ; BAUL , B. ; LEDETI, I. ; VLASE, T. ; MURARIU M.** Evaluation of thermal stability and kinetic of degradation for levodopa in non-isothermal conditions. *J Therm Anal Calorim,* 2018, vol. 131, 1881-1888
- **MALEBARI AM ; KARA A ; KHAYYAT AN ; MOHAMMAD KA ; SERRANO DR.** Development of Advanced 3D-Printed Solid Dosage Pediatric Formulations for HIV Treatment. *Pharmaceuticals,* 2022, vol. 15 (4), 435
- **MATHEW E ; PITZANTI G ; LARRAÑETA E ; LAMPROU DA.** 3D Printing of Pharmaceuticals and Drug Delivery Devices. *Pharmaceutics,* 2020, vol. 12 (3), 266
- **MENDIBIL X ; TENA G ; DUQUE A ; URANGA N ; CAMPANERO MÁ ; ALONSO J.** Direct Powder Extrusion of Paracetamol Loaded Mixtures for 3D Printed Pharmaceutics for Personalized Medicine via Low Temperature Thermal Processing. *Pharmaceutics,* 2021, vol. 13 (6), 907
- **MOORE, J.W. ; H.H. FLANNER.** Mathematical comparison of dissolution profiles. *Pharmaceutical technology,* 1996, vol. 20 (6), 64-74
- **NAGAPUDI, K. ; JONA, J.** *Amorphous Active Pharmaceutical Ingredients in Preclinical Studies: Preparation, Characterization, and Formulation, Current Bioactive Compounds,* 2008, vol. 400, 213-224
- **NORDSTRÖM J ; KLEVAN I ; ALDERBORN G.** A protocol for the classification of powder compression characteristics. *Eur J Pharm Biopharm.,* January 2012, vol. 80 (1), 209-16
- **PANDI P ; BULUSU R ; KOMMINENI N ; KHAN W ; SINGH M.** Amorphous solid dispersions: An update for preparation, characterization, mechanism on bioavailability, stability, regulatory considerations and marketed products. *Int J Pharm.,* 30 August 2020, vol. 586, 119560
- **PITZANTI G ; MATHEW E ; ANDREWS GP ; JONES DS ; LAMPROU DA.** 3D Printing: an appealing technology for the manufacturing of solid oral dosage forms. *J Pharm Pharmacol.,* 10 October 2022, vol. 74 (10), 1427-1449
- **RICE T ; ZANNINI E ; K ARENDT E ; COFFEY A.** A review of polyols - biotechnological production, food applications, regulation, labeling and health effects. *Crit Rev Food Sci Nutr.,* 2020, vol. 60 (12), 2034-2051
- **S'ARI, M. ; BLADE, H. ; COSGROVE, S. ; DRUMMOND-BRYDSON, R. ; HONDOW, N. ; HUGHES L.P. ; BROWN, A.** Characterization of Amorphous Solid Dispersions and Identification of Low Levels of Crystallinity by Transmission Electron Microscopy. *Mol. Pharmaceutics,* 2021, vol. 18 (5), 1905-1919
- **SÁNCHEZ-GUIRALES SA ; JURADO N ; KARA A ; LALATSA A ; SERRANO DR.** Understanding Direct Powder Extrusion for Fabrication of 3D Printed Personalised Medicines: A Case Study for Nifedipine Minitablets. *Pharmaceutics,* 2021, vol. 13 (10), 1583
- **SEOANE-VIAÑO I ; JANUSKAITE P ; ALVAREZ-LORENZO C ; BASIT AW ; GOYANES A.** Semi-solid extrusion 3D printing in drug delivery and biomedicine: Personalised solutions for healthcare challenges. *J Control Release.,* 10 April 2021, vol. 332, 367-389

- **SCHILLING S.U. ; SHAH, N.H. ; MALICK A.W. ; INFELD M.H. ; MCGINITY JW.** Citric acid as a solid-state plasticizer for Eudragit RS PO. *Journal of Pharmacy and Pharmacology,* November 2007, vol. 59 (11), 1493-1500
- **SERRANO DR ; KARA A ; YUSTE I ; LUCIANO FC ; ONGOREN B ; ANAYA BJ ; MOLINA G ; DIEZ L ; RAMIREZ BI ; RAMIREZ IO.** 3D Printing Technologies in Personalized Medicine, Nanomedicines, and Biopharmaceuticals. *Pharmaceutics,* 2023, vol. 15 (2), 313
- **SHI K ; SALVAGE JP ; MANIRUZZAMAN M ; NOKHODCHI A.** Role of release modifiers to modulate drug release from fused deposition modelling (FDM) 3D printed tablets. *Int J Pharm.,* 15 March 2021, vol. 597, 120315
- **SOLANKI NG ; TAHSIN M ; SHAH AV ; SERAJUDDIN ATM.** Formulation of 3D Printed Tablet for Rapid Drug Release by Fused Deposition Modeling: Screening Polymers for Drug Release, Drug-Polymer Miscibility and Printability. *J Pharm Sci.,* January 2018, vol. 107 (1), 390-401
- **SUBRAMANIAN, V.B. ; KONDURU N. ; KATARI N.K. ; DONGALA T. ; GUNDLA R.** A simple high-performance liquid chromatography method development for Carbidopa and Levodopa impurities: Evaluation of risk assessment before method validation by Quality by Design approach. *Separation Science Plus,* 2020, vol. 3 (11-12), 530-539
- **TAGAMI T ; ITO E ; KIDA R ; HIROSE K ; NODA T ; OZEKI T.** 3D printing of gummy drug formulations composed of gelatin and an HPMC-based hydrogel for pediatric use. *Int J Pharm.,* 01 February 2021, vol. 594, 120118
- **VARGHESE R. ; SALVI S. ; SOOD P. ; KARSIYA P. ; KUMAR D.** Recent advancements in additive manufacturing techniques employed in the pharmaceutical industry: A bird's eye view. *Annals of 3D Printed Medicine,* 2022, vol. 8, 100081
- **VERBEECK, R. K. ; MUSUAMBA, F. T.** The revised EMA guideline for the investigation of bioequivalence for immediate release oral formulations with systemic action. *Journal of Pharmacy & Pharmaceutical Sciences,* 2012, vol. 15 (3), 376-388
- **WINDOLF H ; CHAMBERLAIN R ; QUODBACH J.** Predicting Drug Release from 3D Printed Oral Medicines Based on the Surface Area to Volume Ratio of Tablet Geometry. *Pharmaceutics,* 2021, vol. 13 (9), 1453
- **WINDOLF H ; CHAMBERLAIN R ; BREITKREUTZ J ; QUODBACH J.** 3D Printed Mini-Floating-Polypill for Parkinson's Disease: Combination of Levodopa, Benserazide, and Pramipexole in Various Dosing for Personalized Therapy. *Pharmaceutics,* 2022, vol. 14 (5), 931
- **YAN TT ; LV ZF ; TIAN P ; LIN MM ; LIN W ; HUANG SY ; CHEN YZ.** Semi-solid extrusion 3D printing ODFs: an individual drug delivery system for small scale pharmacy. *Drug Dev Ind Pharm.,* April 2020, vol. 46 (4), 531-538
- **YORK, P.** Crystal engineering and particle design for the powder compaction process. *Drug Development and Industrial Pharmacy,* 1992, vol. 18 (6-7), 677-721
- **YU I ; CHEN RK.** A Feasibility Study of an Extrusion-Based Fabrication Process for Personalized Drugs. *Journal of Personalized Medicine,* 2020, vol. 10 (1), 16
- **YU LX ; AMIDON G ; KHAN MA ; HOAG SW ; POLLI J ; RAJU GK ; WOODCOCK J.** Understanding pharmaceutical quality by design. *AAPS J.,* July 2014, vol. 16 (4), 771-83
- **ZHANG J ; VO AQ ; FENG X ; BANDARI S ; REPKA MA.** Pharmaceutical Additive Manufacturing: a Novel Tool for Complex and Personalized Drug Delivery Systems. *AAPS PharmSciTech.,* November 2018, vol. 19 (8), 3388-3402